# EUROPEAN PATENT APPLICATION

(11) **EP 4 772 122 A1**
(43) Date of publication of application: **08.07.2026**
(21) Application number: 24857834.6
(22) Date of filing: 10.05.2024
(51) Int. Cl.: A61B 18/12, A61B 17/29, A61B 17/28, A61B 17/00, A61B 18/14

(54) **INTRACAVITARY SURGICAL INSTRUMENT AND ELECTROCOAGULATION SURGICAL INSTRUMENT**

(30) Priority: 31.08.2023 CN 202311125526
(71) Applicant: Mao, Zhangfan, Wuhan, Hubei 430010 (CN); Mao, Hang, Wuhan, Hubei 430010 (CN)
(72) Inventor: Mao, Zhangfan, Wuhan, Hubei 430010 (CN); Mao, Hang, Wuhan, Hubei 430010 (CN)
(74) Representative: Metida
(86) International application number: PCT/CN2024/092330
(87) International publication number: WO 2025/044285

(57) **Abstract**

An intracavitary surgical instrument comprises an operation component and a driving component that disassembled from each other, thus making full use of multiple wounds of endoscopic surgery, so that the operation component and the rod member may be placed into the cavity from wounds at different positions, reducing the corresponding wound size of the rod member, and improving the recovery speed of patients after surgery. Besides, in the assembly process of the intracavitary surgical instrument, the traction portion may pull the operating component to move in the opposite direction with the driving component, so as to facilitate the assembly of both. Meanwhile, the traction portion may further make the first operating arm and the second operating arm close to each other through the pulling action, so as to realize the electrocoagulation of biological tissues by the first operating arm and the second operating arm, and simplify the operation action.

## Description

The present application claims the priorities to Chinese Patent Application 202311125526.8, titled "ELECTROCOAGULATION SURGICAL INSTRUMENT", which is filed on August 31, 2023, and incorporated herein by reference in its entirety.

### BACKGROUND OF THE DISCLOSURE

### 1. Field of the Disclosure

The present disclosure relates to the field of medical surgical instruments, and particularly to an intracavitary surgical instrument and electrocoagulation surgical instruments.

### 2. Description of the Related Art

In laparoscopic surgery, multiple incisions are made on the human body to facilitate the coordination of tools such as endoscopes, surgical knives, and surgical forceps. For example, a wound on one side can facilitate the migration of tissue within the cavity using surgical forceps, allowing the lesion to be exposed. Thus, the surgical knife can be used to treat the wound from the other side.

In order to insert intracavitary surgical instruments into the human body, if a separate incision is made, it will further increase the patient's pain and slow down the healing process. At the same time, it will also make the space inside the cavity more cramped during surgery. How to reduce the number and size of incisions in intracavitary surgery has become a problem that needs to be solved.

### BRIEF DESCRIPTION OF THE DISCLOSURE

In view of this, embodiments of the present disclosure provide an intracavitary surgical instrument and electrocoagulation surgical instruments, utilizing an electrocoagulation component and a driving component that may be mutually detached, or an operating component and a driving component that may be mutually detached, to reduce the incision size on the insertion side of the driving component and accelerate the healing speed of the incision.

According to a first aspect of the embodiments of the present disclosure, an intracavitary surgical instrument is provided. The intracavitary surgical instrument comprises:
a driving component, including a connecting portion, the connecting portion comprising a hollow rod member, the rod member comprising a connecting end, the connecting end comprising a contact ring surface and an insertion head, the insertion head protruding from the contact ring surface; and
an operating component, including a sheath portion and a moving portion, the sheath portion having a match end surface and a first containing hole, the first containing hole being a through hole, the moving portion comprising a traction portion, a first operating arm, and a second operating arm, the second operating arm being disposed on the sheath portion;
the traction portion is configured such that one end is connected to the first operating arm, and the other end is operably passed through the rod member and pulls the sheath portion to move toward the rod member until the insertion head is detachably inserted through the first containing hole, and the contact ring surface abuts the match end surface, the traction portion being configured to drive the first operating arm to approach the second operating arm.

Further, the traction portion comprises a traction line, the traction line comprising a connecting section and a fixing section, the connecting section being proximate to the first operating arm;
the driving component further comprises a fixing portion, the fixing portion being disposed on a side of the connecting portion away from the operating component and provided with a first duct;
the fixing section passes through the rod member into the first duct and is fixedly connected to the fixing portion, the fixing portion being operably moved toward or away from the connecting portion to drive the first operating arm to move relative to the second operating arm.

Further, the first duct is a through hole;
the traction line further comprises a traction section;
the driving component further comprises a main body;
an end of the connecting portion away from the connecting end is connected to the main body;
the intracavitary surgical instrument has an assembled state, in the assembled state, the traction section extends from an end of the first duct away from the connecting section and pulls the sheath portion to assemble with the connecting end, and the fixing section is located within the first duct.

Further, the rod member is provided with a second duct, the second duct is a through hole;
the main body is provided with a first sliding track, the fixing portion is slidably disposed on the first sliding track, the fixing portion has a first position and a second position within the first sliding track, in the first position, the fixing portion is located at an end of the first sliding track proximate to the sheath portion and the first duct and the second duct extend along the same axis, in the second position, the fixing portion is located at an end of the first sliding track away from the sheath portion, the first operating arm approaches the second operating arm and maintains a predetermined distance.

Further, an axial direction of the first sliding track is parallel to the first duct and the second duct.

Further, a port on a side of the first duct opposite to the second duct has a predetermined distance;
the fixing portion is provided with a guiding tapered surface, a small-diameter end of the guiding tapered surface being connected to a port of the first duct proximate to the second duct.

Further, the traction line is flexible;
the main body has a fourth duct and a containing cavity, the traction line is operably passed through the fourth duct and the first sliding track, the containing cavity is located between the fourth duct and the first sliding track, the fixing portion moves toward the sheath portion, and a region of the containing cavity between the fourth duct and the fixing portion forms an avoidance space;
the traction line extends into the containing cavity, an end of the traction line is placed on the guiding tapered surface and slides from the guiding tapered surface into the first duct.

Further, the fixing portion further comprises:
a positioning member;
a fixing screw; and
a base, provided with the first duct and a side hole, the side hole communicates with the first duct and faces a lateral direction of the first duct, the side hole has a threaded section and a sliding section in an axial direction, the sliding section is adjacent to the first duct, the positioning member is slidably disposed in the sliding section;
the fixing screw is configured to pass through the threaded section and push the positioning member to move into the first duct to fix the fixing section.

Further, the main body is provided with a window, the window penetrates a side wall of one side of the first sliding track and has a length direction parallel to the axial direction of the first sliding track;
a head of the fixing screw extends out from the window, and in the first position and the second position, the fixing screw is moved to edge positions along the length direction of the window.

Further, the positioning member comprises a plurality of connecting handles and a sliding body adapted to the sliding section, the plurality of connecting handles protrude from a side of the sliding body away from the fixing screw;
the fixing portion further comprises:
a plurality of first elastic members; and
a bottom of the side hole is provided with a plurality of first positioning holes, the plurality of first positioning holes are arranged on both sides of the first duct, the plurality of first elastic members are respectively disposed within the plurality of first positioning holes;
the fixing screw is screwed into the threaded section, a tail of the fixing screw pushes the sliding body toward the first duct to clamp the fixing section, and the plurality of connecting handles are respectively inserted into the plurality of first positioning holes and abut the plurality of first elastic members.

Further, when the fixing screw is unscrewed from the threaded section, the first elastic members lift the positioning member.

Further, the first sliding track has a tail port, the tail port is located on a side of the first sliding track away from the sheath portion;
the driving component further comprises a sealing member, the sealing member comprising a limit protrusion, the limit protrusion being provided with a third duct;
the fixing section is located within the first duct, the traction section extends out through the tail port to an exterior of the main body;
the sealing member seals the tail port, the third duct and the first duct extend along the same axis, and at least part of the traction section is retractably passed through the third duct;
in the second position, the limit protrusion abuts the fixing portion.

Further, the intracavitary surgical instrument further comprises:
a puncture needle; and
a coupling soft tube, being a hollow structure and comprising a head portion, a tail portion, and an intermediate region between the head portion and the tail portion;
the coupling soft tube passes through the puncture needle, and the puncture needle is located between the intermediate region and the tail portion, the head portion is configured to couple with the traction line, and the tail portion is configured to drive the traction line through the puncture needle.

Further, the traction portion further comprises a moving head, the moving head being connected to the connecting section and a tail end of the first operating arm;
the sheath portion comprises a second elastic member, a second sliding track, and a stop ring surface, the stop ring surface being located between the second sliding track and the first containing hole, the moving head is slidably disposed within the second sliding track, and the second elastic member abuts between the moving head and the stop ring surface;
in a free state of the traction portion, the second elastic member pushes the first operating arm away from the second operating arm;
in the assembled state, at least part of the connecting end is inserted into the first containing hole.

Further, the insertion head is a puncture head, the puncture head protruding from a central region of the contact ring surface;
the sheath portion further comprises:
an elastic sleeve, disposed on the second sliding track, the second elastic member abuts against the stop ring surface via the elastic sleeve, the elastic sleeve is provided with a tapered hole, a small-diameter end of the tapered hole is away from the stop ring surface, and an inner diameter of at least part of the tapered hole is smaller than an outer diameter of the insertion head;
in the assembled state, the puncture head is simultaneously inserted through the first containing hole and the tapered hole until the contact ring surface abuts the match end surface.

Further, the operating component further comprises a connecting sleeve, the connecting sleeve comprising a plurality of positioning elastic sheets, the plurality of positioning elastic sheets having elastic bending amounts toward an exterior of the connecting sleeve;
the connecting sleeve is disposed in the first containing hole, the insertion head is inserted into the connecting sleeve, and the plurality of positioning elastic sheets abut against a lateral side of the insertion head.

Further, the rod member comprises a first extension section and a second extension section in a length direction, a diameter of the first extension section is less than 3 millimeters, and the diameter of the first extension section is smaller than a diameter of the second extension section.

The embodiments of the present disclosure further provide an electrocoagulation surgical instrument. The electrocoagulation surgical instrument comprises:
a driving component, including a connecting portion, the connecting portion comprising a hollow rod member, the rod member comprising a connecting end; and
an electrocoagulation component, including a sheath portion and a moving portion, the moving portion comprising a traction portion and a pair of first and second operating arms, the second operating arm being disposed on the sheath portion;
the traction portion is configured such that one end is connected to the first operating arm and the traction portion and the first operating arm form a first electrical pathway, the other end is operably passed through the rod member and pulls the sheath portion to move toward the rod member until the connecting end is detachably connected to the sheath portion, so that the rod member, the sheath portion, and the second operating arm form a second electrical pathway, the traction portion being configured to drive the first operating arm to approach the second operating arm, thereby enabling the first electrical pathway to conduct with the second electrical pathway through biological tissue.

Further, the traction portion comprises a traction line, the traction line comprising a connecting section and a fixing section, the connecting section being proximate to the first operating arm;
the driving component further comprises a fixing portion, the fixing portion being disposed on a side of the connecting portion away from the electrocoagulation component and provided with a first duct;
the fixing section passes through the rod member into the first duct and is fixedly connected to the fixing portion, the fixing portion being operably moved toward or away from the connecting portion to drive the first operating arm to move relative to the second operating arm.

Further, the first duct is a through hole;
the traction line further comprises a traction section;
the driving component further comprises a main body;
an end of the connecting portion away from the connecting end is connected to the main body;
the electrocoagulation surgical instrument has an assembled state, in the assembled state, the traction section extends from an end of the first duct away from the connecting section and pulls the sheath portion to assemble with the connecting end, and the fixing section is located within the first duct.

Further, the rod member is provided with a second duct, the second duct being a through hole;
the main body is provided with a first sliding track, the fixing portion is slidably disposed on the first sliding track, the fixing portion has a first position and a second position within the first sliding track, in the first position, the fixing portion is located at an end of the first sliding track proximate to the sheath portion and the first duct and the second duct extend along the same axis, in the second position, the fixing portion is located at an end of the first sliding track away from the sheath portion, the first operating arm approaches the second operating arm and maintains a predetermined distance.

Further, a port on a side of the first duct opposite to the second duct has a predetermined distance;
the fixing portion is provided with a guiding tapered surface, a small-diameter end of the guiding tapered surface being connected to a port of the first duct proximate to the second duct.

Further, the fixing portion further comprises:
a positioning member;
a fixing screw; and
a base, provided with the first duct and a side hole, the side hole communicates with the first duct and faces a lateral direction of the first duct, the side hole has a threaded section and a sliding section in an axial direction, the sliding section is adjacent to the first duct, the positioning member is slidably disposed in the sliding section;
the fixing screw is configured to pass through the threaded section and push the positioning member to move into the first duct to fix the fixing section.

Further, the main body is provided with a window, the window penetrates a side wall of one side of the first sliding track and has a length direction parallel to the axial direction of the first sliding track;
a head of the fixing screw extends out from the window, and in the first position and the second position, the fixing screw is moved to edge positions along the length direction of the window.

Further, the base and the fixing screw are insulating members;
the positioning member comprises a plurality of connecting handles and a sliding body adapted to the sliding section, the plurality of connecting handles protrude from a side of the sliding body away from the fixing screw;
the fixing portion further comprises:
a plurality of elastic members; and
a first electrical contact member, disposed on the base and comprising an electrical contact surface, the electrical contact surface protruding into the first duct;
a bottom of the side hole is provided with a plurality of first positioning holes, the plurality of first positioning holes are arranged on both sides of the first duct, the plurality of elastic members are respectively disposed within the plurality of first positioning holes;
the fixing screw is screwed into the threaded section, a tail of the fixing screw pushes the sliding body toward the electrical contact surface to clamp the fixing section, and the first electrical contact member is electrically connected to the fixing section, while the plurality of connecting handles are respectively inserted into the plurality of first positioning holes and abut the plurality of elastic members.

Further, the first sliding track has a tail port, the tail port is located on a side of the first sliding track away from the sheath portion;
the driving component further comprises a sealing member, the sealing member being an insulating member and comprising a limit protrusion, the limit protrusion being provided with a third duct;
the fixing section is located within the first duct, the traction section extends out through the tail port to an exterior of the main body;
the sealing member seals the tail port, the third duct and the first duct extend along the same axis, and at least part of the traction section is retractably passed through the third duct;
in the second position, the limit protrusion abuts the fixing portion.

Further, the connecting portion comprises a first connector, the first connector being disposed on an end of the rod member away from the connecting end;
the main body comprises:
a containing portion, provided with the first sliding track; and
a second connector, protruding from a side of the containing portion and provided with a fourth duct, the fourth duct communicates with the first sliding track;
the driving component further comprises a first wire;
the first connector and the second connector are in a connected state, the fourth duct and the first duct extend along the same axis, and one end of the first wire is electrically connected to the first connector, and the other end extends to an exterior of the main body.

Further, the driving component further comprises:
a second electrical contact member, provided with a central through hole;
the second connector is provided with a fifth duct, the fifth duct communicates with the first sliding track;
the first connector and the second connector are paired and in a connected state, the second electrical contact member abuts between an end surface of the rod member and the second connector, and the central through hole aligns with the first duct and the fourth duct, the first wire passes through the first sliding track and the fifth duct to connect to the second electrical contact member.

Further, the traction portion further comprises a moving head, the moving head being connected to the connecting section and a tail end of the first operating arm;
the sheath portion comprises an elastic member, a second sliding track, a stop ring surface, and a first containing hole, the first containing hole being a through hole, the stop ring surface being located between the second sliding track and the first containing hole, the moving head is slidably disposed within the second sliding track, and the elastic member abuts between the moving head and the stop ring surface;
in a free state of the traction portion, the elastic member pushes the first operating arm away from the second operating arm;
in the assembled state, at least part of the connecting end is inserted into the first containing hole.

Further, the connecting end comprises a contact ring surface and a puncture head, the puncture head protruding from a central region of the contact ring surface;
the sheath portion further comprises:
an elastic sleeve, disposed on the second sliding track, the elastic member abuts against the stop ring surface via the elastic sleeve, the elastic sleeve is provided with a tapered hole, a small-diameter end of the tapered hole is away from the stop ring surface, and an inner diameter of at least part of the tapered hole is smaller than an outer diameter of the puncture head;
in the assembled state, the puncture head is simultaneously inserted through the first containing hole and the tapered hole until the contact ring surface abuts the sheath portion.

Further, the rod member is a conductive member and comprises a transition segment and an opposite end, the transition segment being located between the connecting end and the opposite end;
the connecting portion further comprises a first insulating coating layer, the first insulating coating layer coats the transition segment, and the connecting end is exposed outside the first insulating coating layer;
the sheath portion further comprises a conductive body and a second insulating coating layer covering partial outer regions of the conductive body, the second operating arm is disposed on the conductive body, the conductive body has a match end surface;
the match end surface is exposed outside the second insulating coating layer and abuts the contact ring surface.

Further, the sheath portion further comprises:
a conductive body, provided with a second containing hole, the stop ring surface, and the first containing hole, the second containing hole has an opening end away from the first containing hole; and
an insulating liner, comprising a flanged edge and a tubular body, an inner wall of the tubular body forms the second sliding track;
the tubular body is disposed within the second containing hole, the flanged edge is clamped at the opening end.

Further, the moving portion further comprises an insulating spacer block, the insulating spacer block being disposed between the first operating arm and the second operating arm;
the flanged edge comprises two pieces extending in opposite directions;
the conductive body comprises two support arms, the two support arms protrude from the opening end and are staggered with the two flanged edges;
the insulating spacer block has a first hinge hole and a second hinge hole, the second operating arm has a third hinge hole and a fourth hinge hole, the first operating arm has a fifth hinge hole and a sixth hinge hole, the support arms have a seventh hinge hole, the moving head has an eighth hinge hole;
the first hinge hole and the third hinge hole are simultaneously hinged with the seventh hinge hole, the second hinge hole and the fourth hinge hole are simultaneously hinged with the fifth hinge hole, the eighth hinge hole is hinged with the sixth hinge hole, and the two flanged edges correspond to the first operating arm and the second operating arm, respectively.

Further, the traction line further comprises:
a conductive core and a third insulating coating layer, the third insulating coating layer coats the conductive core and corresponding regions of the rod member, and the conductive core and corresponding regions of the fixing portion are exposed outside the third insulating coating layer.

The embodiments of the present disclosure further provide another electrocoagulation surgical instrument. The electrocoagulation surgical instrument comprises:
a driving component, including a connecting portion, the connecting portion comprising a hollow rod member, the rod member comprising a connecting end; and
an electrocoagulation component, including a sheath portion, a traction portion, and a moving portion disposed on the sheath portion, the moving portion comprising a first operating arm and a second operating arm;
the traction portion is configured such that one end is connected to the moving portion, the other end is operably passed through the rod member and pulls the sheath portion to move toward the rod member until the connecting end is detachably connected to the sheath portion, the traction portion being configured to drive the first operating arm to approach the second operating arm;
the moving portion is configured such that the first operating arm and the second operating arm are conductively connected through the sheath portion and the rod member, and/or the first operating arm and the second operating arm are conductively connected with the traction portion.

Further, the traction portion is configured such that one end is connected to the first operating arm, the second operating arm is disposed on the sheath portion, the first operating arm and the second operating arm are pivotally connected and in electrical contact;
the traction portion comprises a traction line, the traction line comprising a connecting section and a fixing section, the connecting section being proximate to the first operating arm;
the driving component further comprises a fixing portion, the fixing portion being disposed on a side of the connecting portion away from the electrocoagulation component and provided with a first duct;
the fixing section passes through the rod member and extends into the first duct to be fixedly connected to the fixing portion, the fixing portion being operably moved toward or away from the connecting portion to drive the first operating arm to move relative to the second operating arm.

Further, the first duct is a through hole;
the traction line further comprises a traction section;
the driving component further comprises a main body;
an end of the connecting portion away from the connecting end is connected to the main body;
the electrocoagulation surgical instrument has an assembled state, in the assembled state, the traction section extends from an end of the first duct away from the connecting section and pulls the sheath portion to assemble with the connecting end, and the fixing section is located within the first duct.

Further, the connecting portion comprises a first connector, the first connector being disposed on an end of the rod member away from the connecting end, the rod member is provided with a second duct, the second duct being a through hole;
the main body comprises:
a containing portion, provided with a first sliding track, the fixing portion is slidably disposed on the first sliding track, the fixing portion has a first position and a second position within the first sliding track, in the first position, the fixing portion is located at an end of the first sliding track proximate to the sheath portion and the first duct and the second duct extend along the same axis, in the second position, the fixing portion is located at an end of the first sliding track away from the sheath portion, the first operating arm approaches the second operating arm; and
a second connector, being a conductive member and provided with a fourth duct, the traction line is operably passed through the fourth duct and the first sliding track;
the first connector and the second connector are fixedly connected, and the second connector is conductively connected to the sheath portion through the rod member.

Further, the second connector is provided with a positioning groove, the positioning groove is recessed from an outer surface of the second connector toward the fourth duct;
the driving component further comprises a housing portion and a conductive ring, the conductive ring is disposed in the positioning groove and electrically connected to the second connector, at least part of the second connector is disposed inside the housing portion, the housing portion being an insulating member.

Further, the driving component further comprises a conductive arm, one end of the conductive arm is connected to the conductive ring, and the other end extends to an exterior of the housing portion.

Further, the traction portion further comprises a moving head, the moving head being connected to the connecting section and a tail end of the first operating arm;
the sheath portion comprises a second elastic member, a second sliding track, a stop ring surface, and a first containing hole, the first containing hole being a through hole, the stop ring surface being located between the second sliding track and the first containing hole, the moving head is slidably disposed within the second sliding track, and the second elastic member abuts between the moving head and the stop ring surface;
in a free state of the traction portion, the second elastic member pushes the first operating arm away from the second operating arm;
in the assembled state, at least part of the connecting end is inserted into the first containing hole.

Further, the connecting end comprises a contact ring surface and an insertion head, the insertion head protruding from a central region of the contact ring surface;
the sheath portion further comprises:
an elastic sleeve, disposed on the second sliding track, the second elastic member abuts against the stop ring surface via the elastic sleeve, the elastic sleeve is provided with a tapered hole, a small-diameter end of the tapered hole is away from the stop ring surface, and an inner diameter of at least part of the tapered hole is smaller than an outer diameter of the insertion head;
in the assembled state, the insertion head is simultaneously inserted through the first containing hole and the tapered hole until the contact ring surface abuts the sheath portion.

Further, the rod member is a conductive member and comprises a transition segment and an opposite end, the transition segment being located between the connecting end and the opposite end, the first connector is disposed on the opposite end;
the connecting portion further comprises a first insulating coating layer, the first insulating coating layer coats the transition segment, and the connecting end is exposed outside the first insulating coating layer;
the sheath portion further comprises a conductive body and a second insulating coating layer covering partial outer regions of the conductive body, the second operating arm is disposed on the conductive body, the conductive body has a match end surface;
the match end surface is exposed outside the second insulating coating layer and abuts the contact ring surface.

The intracavitary surgical instrument of the embodiments of the present disclosure is configured with an operating component and a driving component that may be disassembled from each other, and the electrocoagulation surgical instrument is configured with an electrocoagulation component and a driving component that may be disassembled from each other. This fully utilizes multiple incisions in laparoscopic surgery, allowing the operating component or the electrocoagulation component and the rod member to be inserted into the cavity through incisions at different positions, thereby reducing the incision size corresponding to the rod member and improving postoperative recovery speed. On the other hand, during the component of the electrocoagulation surgical instrument or the intracavitary surgical instrument, the traction portion may pull the electrocoagulation component or the operating component and the driving component toward each other for convenient component. Simultaneously, the traction portion further drives the first operating arm and the second operating arm to approach each other via traction, enabling electrocoagulation operations on biological tissue, thereby simplifying surgical maneuvers.

### BRIEF DESCRIPTION OF THE DRAWINGS

The above and other objectives, features, and advantages of the present disclosure will become clearer through the following description of embodiments of the present disclosure with reference to the accompanying drawings:
Fig. 1 is a schematic diagram of the structure of an intracavitary surgical instrument in some embodiments of the present disclosure;
Fig. 2 is a schematic diagram of the structure of the intracavitary surgical instrument in other embodiments of the present disclosure;
Fig. 3 is an exploded schematic diagram of a driving component according to an embodiment of the present disclosure;
Fig. 4 is an exploded schematic diagram of the operating component of the embodiment of the present disclosure;
Fig. 5 is a schematic sectional view of the operating component in some embodiments of the present disclosure;
Fig. 6 is a partial sectional schematic diagram of a driving component according to an embodiment of the present disclosure;
Fig. 7 is a schematic diagram of partial explosion of the driving component in some embodiments of the present disclosure;
Fig. 8 is a schematic sectional view of an intracavitary surgical instrument in some embodiments of the present disclosure;
Fig. 9 is a schematic diagram of the structure of a conductive body and a connecting end according to an embodiment of the present disclosure;
Fig. 10 is a schematic diagram of the structure of a base according to an embodiment of the present disclosure;
Fig. 11 is a schematic diagram of the structure of a positioning member according to an embodiment of the present disclosure;
Fig. 12 is a schematic diagram of the workflow of an electrocoagulation surgical instrument according to an embodiment of the present disclosure;
Fig. 13 is a schematic sectional view of an electrocoagulation component in other embodiments of the present disclosure;
Fig. 14 is a schematic diagram of partial explosion of the driving component in other embodiments of the present disclosure;
Fig. 15 is an exploded schematic diagram of a conductive ring, a conductive arm, and a second connector of the embodiment of the present disclosure;
Fig. 16 is a schematic diagram of the structure of a connecting sleeve according to an embodiment of the present disclosure.

### DETAILED DESCRIPTION OF EMBODIMENTS OF THE DISCLOSURE

The following describes the present disclosure based on embodiments, but the present disclosure is not limited to these embodiments. In the following detailed description of the present disclosure, specific details are described in detail. For those skilled in the art, the present disclosure can be fully understood without the description of these detailed parts. In order to avoid confusion about the essence of the present disclosure, the well-known methods, processes, procedures, components, and circuits have not been described in detail.

In addition, ordinary technical personnel in this field should understand that the accompanying drawings provided here are for illustrative purposes and may not necessarily be drawn to scale.

Unless explicitly required by the context, words such as "including" and "containing" in the entire application document should be interpreted as containing rather than exclusive or exhaustive; that is to say, it means "including but not limited to".

In the description of the present disclosure, it should be understood that the terms "first", "second", etc. are only used for descriptive purposes and cannot be understood as indicating or implying relative importance. In addition, in the description of the present disclosure, unless otherwise specified, the meaning of "multiple" refers to two or more.

Unless otherwise specified and limited, the terms "installation", "connection", "connection", "fixation", etc. should be broadly understood, for example, they can be fixed connections, detachable connections, or integrated; It can be directly connected or indirectly connected through an intermediate medium, and can be a connection within two components or an interaction relationship between two components, unless otherwise specified. For ordinary technical personnel in this field, the specific meanings of the above terms in the present disclosure can be understood according to the specific situation.

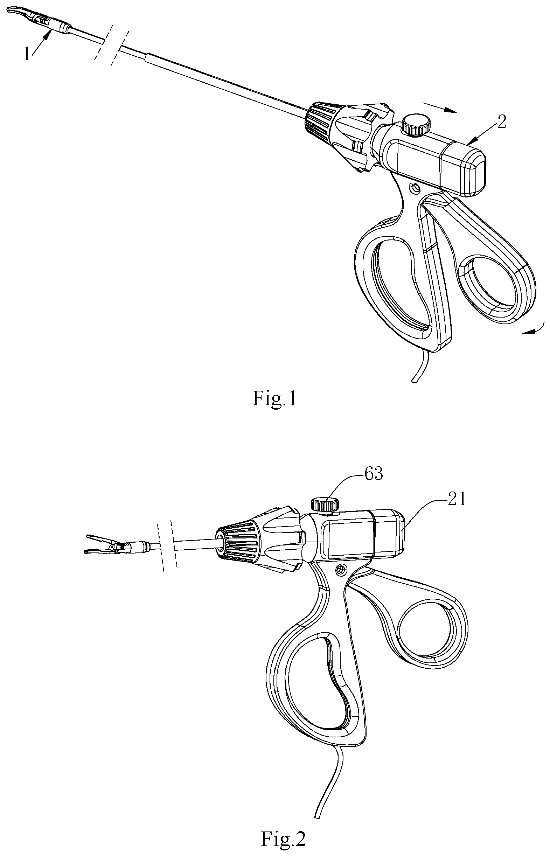

Fig. 1 and Fig. 2 are schematic diagrams of the structure of an intracavitary surgical instrument in different embodiments of this embodiment. This intracavitary surgical instrument may be used as an electrocoagulation surgical instrument. The electrocoagulation surgical instrument in Fig. 2 is in an installed state, with the first operating arm 51 and the second operating arm 52 in Fig. 2 moving away from each other. The electrocoagulation surgical instrument in Fig. 1 is in a discharge state, with the first operating arm 51 and the second operating arm 52 approaching each other and conducting through the hemostatic position.

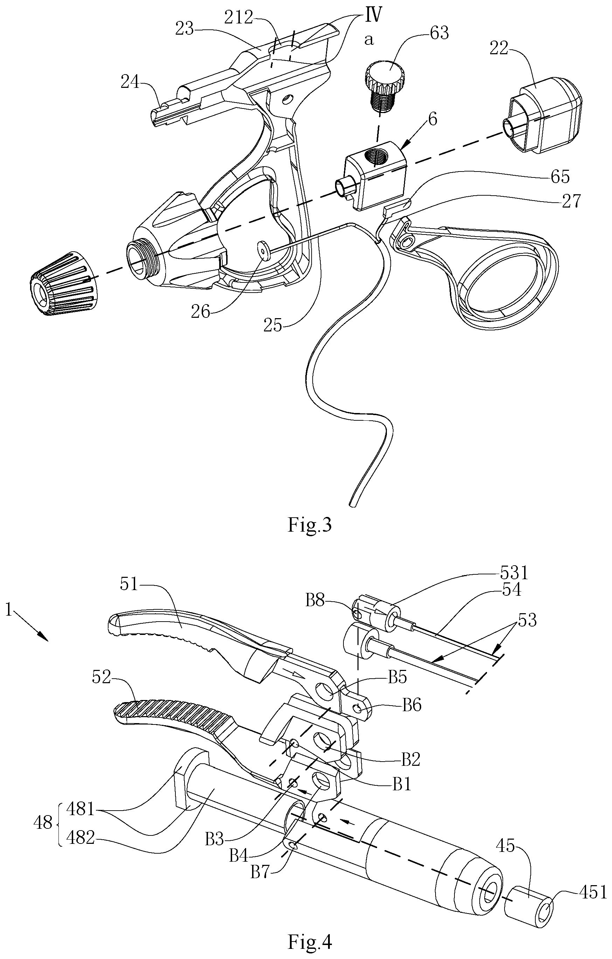

Fig. 3 is an exploded schematic diagram of driving component 2. The connecting portion 3 is not shown in Fig. 3.

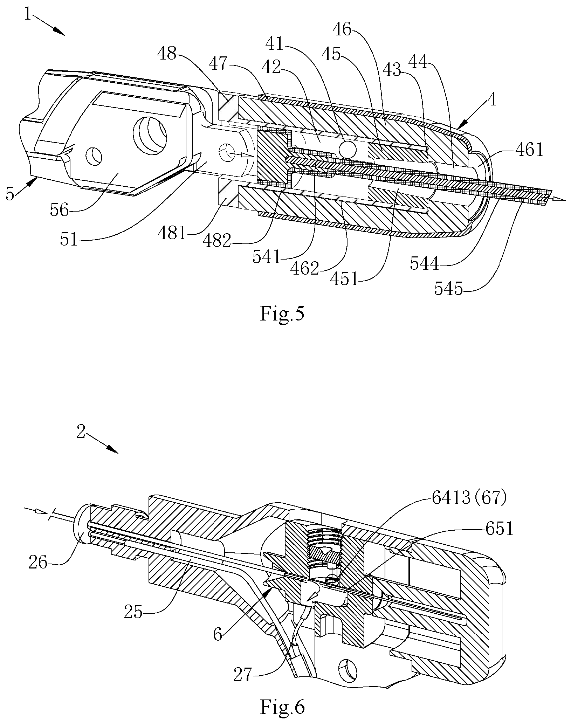

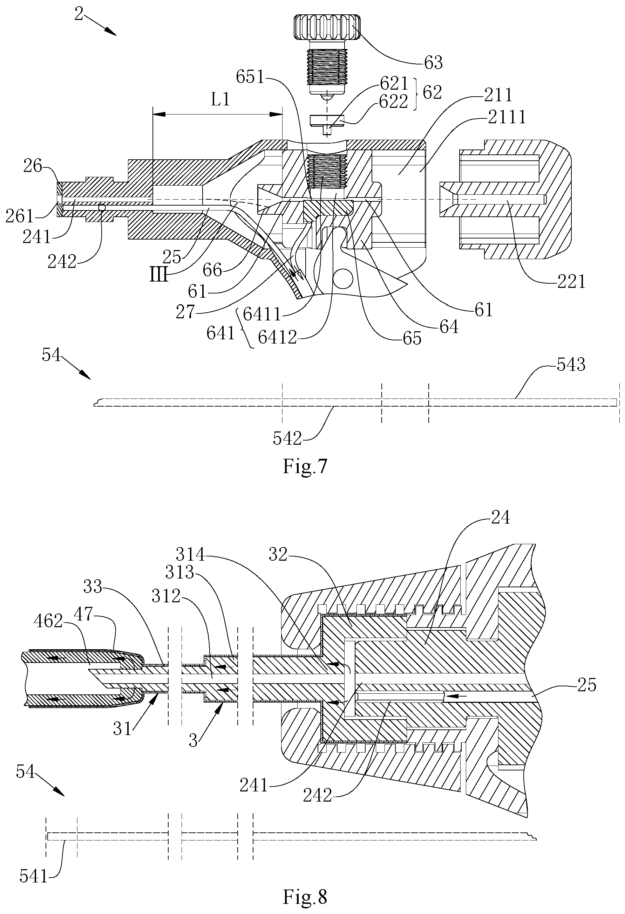

Fig. 4 and Fig. 5 are exploded and sectional diagrams of an operating component. This operating component may be used as an electrocoagulation component 1. Fig. 6 and Fig. 7 are partial sectional and exploded schematic diagrams of the driving component 2. Fig. 8 is a schematic sectional view of an intracavitary surgical instrument, which may be used as an electrocoagulation surgical instrument. The traction portion 53 is not shown in Fig. 8. Meanwhile, in Fig. 7 and Fig. 8, the corresponding relationship between each area on the traction line 54 and the driving component 2 and the sheath portion 4 is further shown.

Specifically, as shown in Figs. 4 to 8, a specific current flow direction is indicated by arrows. Among them, the solid arrow represents the direction of current fed into the second operating arm 52, and the hollow arrow represents the direction of current flowing out of the first operating arm 51.

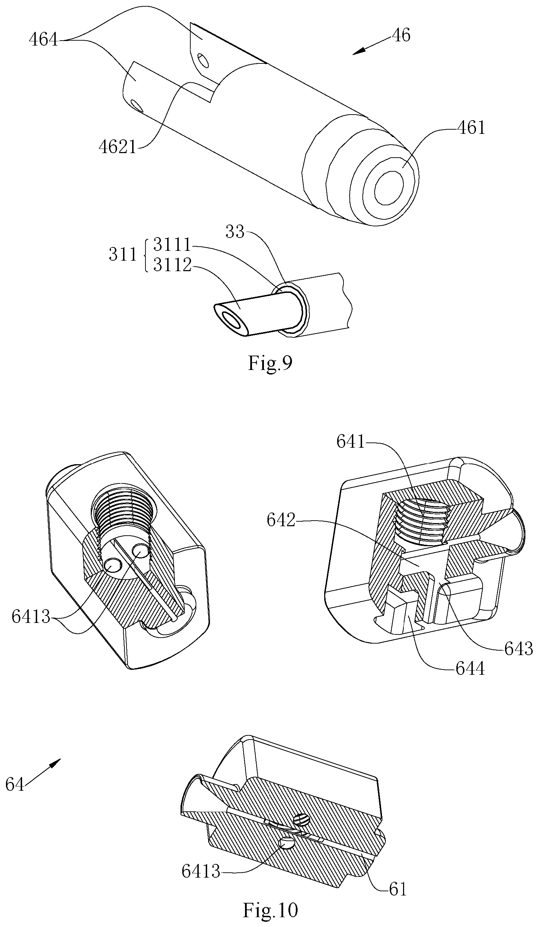

Fig. 9 is a schematic diagram of the structure of a conductive body 46 and a connecting end 311. The outline of the connecting end 311 is shown with a thick solid line in Fig. 9.

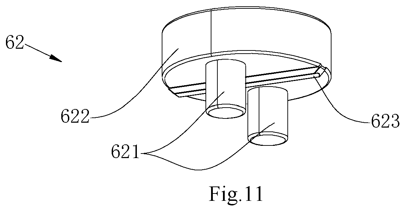

Fig. 10 and Fig. 11 are schematic diagrams of the structure of a base 64 and a positioning member 62.

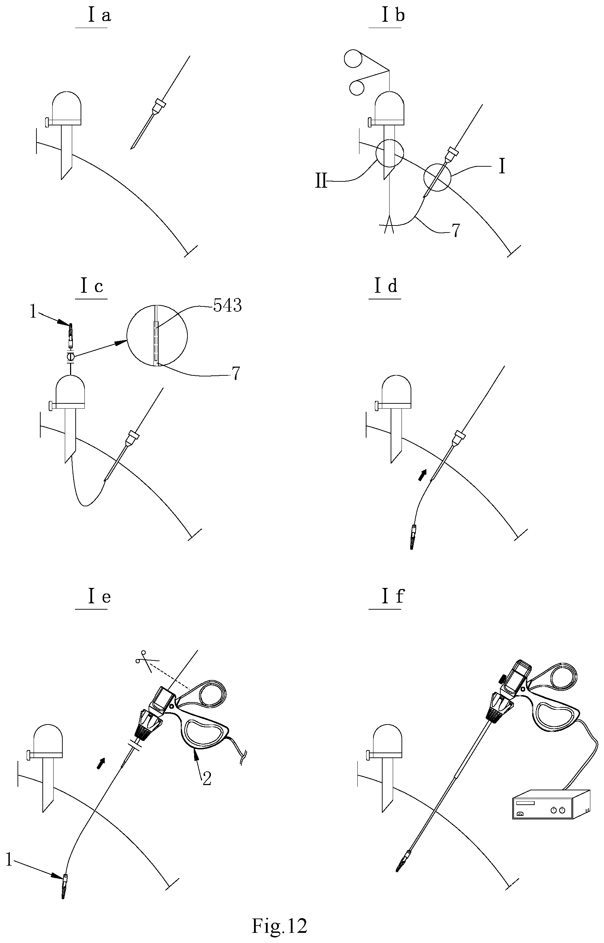

Fig. 12 is a schematic diagram of the workflow of the electrocoagulation surgical instrument in this embodiment. The different stages of surgery from state Ia to state If in Fig. 12. Among them, in state Ib, the puncture needle and puncture sheath are inserted into the cavity through the first and second incisions on region I and region II, respectively. The size of the first incision is smaller than the size of the second incision. In states Ib and Ic, the coupling soft tube 7 inserted into the cavity by the puncture needle is also shown.

In some embodiments, as shown in Figs. 1 to 8, the electrocoagulation surgical instrument in this embodiment includes an electrocoagulation component 1 and a driving component 2. The driving component 2 comprises a connecting portion 3, the connecting portion 3 comprises a hollow rod member 31, and the rod member 31 comprises a connecting end 311. The electrocoagulation component 1 includes a sheath portion 4 and a moving portion 5. The moving portion 5 includes a traction portion 53 and a first operating arm 51 and a second operating arm 52, and the second operating arm 52 is arranged on the sheath portion 4. At the same time, the traction portion 53 is configured that one end is connected with the first operating arm 51, and the traction portion 53 forms a first electrical path with the first operating arm 51. The other end may operably pass through the rod member 31, and pull the sheath portion 4 to move towards the rod member 31, until the connecting end 311 is detachably connected with the sheath portion 4, so that the rod member 31 forms a second electrical path with the sheath portion 4 and the second operating arm 52. The traction portion 53 is used to drive the first operating arm 51 close to the second operating arm 52, so that the first electrical path is connected with the second electrical path through biological tissue.

Specifically, as shown in State Ic in Fig. 1 and Fig. 12, the pulling operation of the traction portion 53 in this embodiment may be divided into three stages. In the first stage, the electrocoagulation component 1 and the driving component 2 may be assembled together through the traction portion 53. In the second stage, the first operating arm 51 and the second operating arm 52 may be driven close to each other by continuing to pull the traction portion 53 to clamp biological tissue. During this process, the electrocoagulation surgical instrument may be used in conjunction with surgical knives and other tools to treat lesions. In the third stage, the first operating arm 51 and the second operating arm 52 may be butted on both sides of the hemostatic position, and the coagulation current may be applied to the hemostatic position through the power supply equipment electrically connected with the first and second electrical paths (as shown in the state If in Fig. 12).

Optionally, the first operating arm 51 and the second operating arm 52 in this embodiment may be configured in the form of a pliers component, a scissors component, a forceps component, or the like. Technicians in this field c may choose based on the actual operation of the surgery. Taking the pliers component as an example, the first operating arm 51 and the second operating arm 52 in this embodiment are configured as two jaws to clamp, pull and apply coagulation current to biological tissues through the proximity of the two jaws. Taking the scissors component as an example, the first operating arm 51 and the second operating arm 52 are configured as two scissors arms, which may perform electrocoagulation while cutting biological tissues.

It is easy to understand that in the application text with the public number CN110545736A, the operating device and hollow needle are respectively inserted into the cavity from the first wound and the second wound, and further controlled to clamp the lesion and pull it in a direction roughly perpendicular to the sheath, in order to expose the lesion area for the doctor's surgical operation.

In this embodiment, the second wound may be used to place the electrocoagulation component 1 into the cavity, the first wound may be used to insert part of the rod member 31 into the cavity, and the surgical tool may be used to insert the traction portion 53 into the rod member 31, so that the operator may easily pull the traction portion 53. Or after the electrocoagulation component 1 is placed in the human body, the tail of the traction portion 53 is led out to the outside of the human body through the puncture needle through the surgical tool, and the traction portion 53 is threaded into the rod member 31 outside the human body.

As a result, the size of the first incision is reduced. When the diameter of the puncture needle and rod member 31 is less than 3 millimeters, the first wound will be closed after the puncture needle and rod member 31 are pulled out. Without the need for suturing, it reduces the damage caused by surgery to the human body.

In some embodiments, as shown in State If in Fig. 8 and Fig. 12, the rod member 31 includes a first extension section and a second extension section in the length direction. The diameter of the first extension section is configured to be less than 3 millimeters, and the diameter of the first extension section is smaller than that of the second extension section.

In intracavitary surgery, a portion of the first extension section in this embodiment is inserted into the cavity, while the second extension section is left on the outside of the human body. Thus, the overall length of the rod member piece 31 may be increased by using the second extension section to facilitate the operation of the operator. At the same time, the second extension section may also increase the mechanical strength of the rod member 31, reduce or even avoid the bending of the rod member 31 during the operation.

To sum up, the electrocoagulation surgical instrument of this embodiment uses the traction portion 53 and the first operating arm 51 to form a first electrical path, and uses the sheath portion 4 and the second operating arm 52 to form a second electrical path to apply coagulation current to biological tissues. Therefore, on the one hand, the electrocoagulation surgical instrument is configured as detachable and spliced electrocoagulation component 1 and driving component 2, which makes full use of multiple wounds of endoscopic surgery, so that the electrocoagulation component 1 and rod member 31 may be placed into the cavity from different wounds, reducing the corresponding wound size of rod member 31, and improving the recovery speed of patients after surgery. On the other hand, during the assembly process of the electrocoagulation component 1 and the driving component 2, the traction portion 53 may pull the electrocoagulation component 1 and the driving component to move in opposite directions, so as to facilitate the assembly of the two. At the same time, the traction portion 53 may further make the first operating arm 51 and the second operating arm 52 close to each other through the pulling action, so as to realize the electrocoagulation of biological tissues by the first operating arm 51 and the second operating arm 52, and simplify the operation of surgery.

In some embodiments, as shown in Figs. 1 to 4, the first operating arm 51 and the second operating arm 52 in this embodiment may be configured to perform surgical operations such as clamping, traction, suture or shearing on biological tissues. When the first operating arm 51 and the second operating arm 52 form a clamp component, the first operating arm 51 and the second operating arm 52 in this embodiment are configured as two clamp heads to clamp or pull biological tissues through the close action of the two clamp heads. When the first operating arm 51 and the second operating arm 52 form a scissors component, the first operating arm 51 and the second operating arm 52 in this embodiment are configured as two scissors arms, and the two scissors arms may cut biological tissue to separate the biological tissue from the human body. When the first operating arm 51 and the second operating arm 52 form a forceps component, the first operating arm 51 and the second operating arm 52 in this embodiment are two forceps rods, and the ends of the first operating arm 51 and the second operating arm 52 are forceps heads. Two tweezer heads may be used to grip suture needles for easy suturing of biological tissues.

In some embodiments, as shown in Figs. 1-8, the traction portion 53 includes a traction line 54, the traction line 54 includes a connecting section 541 and a fixing section 542, and the connecting section 541 is close to the first operating arm 51. The driving component 2 also includes a fixing portion 6, which is arranged on the side of the connecting part 3 away from the electrocoagulation component 1 and is provided with a first duct 61. The fixing section 542 passes through the rod member 31 to the first duct 61 and is fixedly connected with the fixing portion 6, which may be operably close to or far from the connecting portion 3 to drive the first operating arm 51 to move relative to the second operating arm 52.

In this embodiment, the traction line 54 is flexible, so that the electrocoagulation component 1 may be placed into the cavity through the first wound. At the same time, in the first stage, the second stage and the third stage, the traction line 54 may provide the pulling force required for the proximity of the electrocoagulation component 1 and the driving component 2, and the proximity of the first operating arm 51 and the second operating arm 52. At the same time, it may also be used as a wire to guide the solidification current.

Optionally, as shown in Fig. 12, the electrocoagulation surgical instrument also includes a coupling soft tube 7. The coupling soft tube 7 has a hollow structure and certain elastic force. In this embodiment, the coupling soft tube 7 may be inserted into the human body through the puncture needle. Insert the clamping tool into the cavity from the puncture sleeve to clamp the coupling soft tube 7 and bring it out to the outside of the human body through the second wound. In this form, the middle area of the coupling soft tube 7 is located in the cavity. That is to say, the tail did not penetrate into the puncture needle. After the head of the coupling soft tube 7 is sleeved with the traction line 54, pull the tail of the coupling soft tube 7 to bring the electrocoagulation component 1 into the cavity, and continue to pull the coupling soft tube 7, so that the fixing section 542 of the traction line 54 is led out to the outside of the human body through the puncture needle. Then, take down the puncture needle, separate the coupling soft tube 7 from the traction line 54, and thread the traction line 54 into the rod member 31. Finally, pull the traction line 54 to connect the sheath portion 4 with the connecting end 311 of the rod member 31. In this process, the connecting end 311 will be inserted into the cavity through the first wound.

In some embodiments, as shown in Figs. 1 to 8, the first duct 61 is a through hole. The traction line 54 also includes a traction section 543. The driving component 2 also includes a main body 21. The end of the connecting portion 3 far from the connecting end 311 is connected with the main body 21. The electrocoagulation surgical instrument is in an assembled state. In the splicing state, the traction section 543 extends from the end of the first duct 61 far from the connecting section 541, and pulls the sheath portion 4 to splice with the connecting end 311, and the fixing section 542 is located in the first duct 61.

Therefore, on the one hand, the traction section 543 in this embodiment is used for the operator to pull the electrocoagulation component 1, so that the electrocoagulation component 1 and the driving component are mutually matched. On the other hand, when the sheath portion 4 and the connecting end 311 are spliced together, it is ensured that the fixing section 542 is just located in the first duct 61. So that the operator may fix the fixing section 542 located in the first duct 61 through the fixing section 6. On the other hand, the coupling soft tube 7 may be sleeved on the traction section 543, so that the coupling soft tube 7 may lead the traction line 54 out to the outside of the human body through the first wound.

In some embodiments, as shown in Figs. 1-8, the rod member 31 is provided with a second duct 312, and the second duct 312 is a through hole. The main body 21 is provided with a first sliding track 211, and the fixing portion 6 is slidably arranged on the first sliding track 211. The fixing portion 6 has a first position and a second position in the first sliding track 211. In the first position, the fixing portion 6 is located at one end of the first sliding track 211 near the sheath part 4, and the first duct 61 and the second duct 312 extend along the same axis. In the second position, the fixing portion 6 is located at the end of the first sliding track 211 away from the sheath part 4, and the first operating arm 51 approaches the second operating arm 52 and maintains a predetermined distance.

When the fixing portion 6 is moved from the first position to the second position, the traction line 54 may be pulled so that the first operating arm 51 and the second operating arm 52 are close to each other to clamp the biological tissue. At the same time, after the coagulation current is applied to the electrocoagulation surgical instrument, the fixing portion 6 may also be moved from the second position to the first position to release the clamping of biological tissue by the first operating arm 51 and the second operating arm 52.

Optionally, the axial direction of the first sliding track 211 is parallel to the first duct 61 and the second duct 312. When the traction section 543 penetrates into the main body 21 through the rod piece 31, it may be ensured that the traction section 543 moves directly into the second duct 312. It reduces or even avoids the situation that traction line 54 cannot penetrate into first duct 61 and second duct 312 at the same time.

In some embodiments, as shown in Fig. 7, the ports on the opposite side of the first duct 61 and the second duct 312 have a predetermined distance (distance L1). The fixing portion 6 is provided with a guiding tapered surface 66, and the small diameter end of the guiding tapered surface 66 is connected with the port of the first duct 61 near the second duct 312.

It is easy to understand that in this embodiment, a certain distance is kept between the first duct 61 and the second duct 312, which may be used to accommodate the first wire 25, the second wire 27 or other parts, so as to avoid the internal space of the main body 21 being too limited. For example, as shown in Fig. 7, a holding cavity is arranged between the fourth duct 241 and the first sliding track 211. When the fixing portion 6 moves in the direction of the sheath portion 4, it will touch the first wire 25, and the holding cavity is used to provide an avoidance space when the first wire 25 is bent to avoid pulling the first wire 25.

However, when the traction line 54 extends from the first duct 61 to the inside of the main body 21, the flexibility of the traction line 54 will make the traction line 54 bend to one side (as shown by dotted line III in Fig. 7). For this reason, in this embodiment, the guiding tapered surface 66 may guide the end of the traction line 54, especially when the traction line 54 is soft, to ensure that the traction line 54 may pass through the first duct 61 and the second duct 312.

In some embodiments, as shown in Figs. 6 to 7, the fixing portion 6 also includes a positioning member 62, a fixing screw 63 and a base 64. The base 64 is provided with a first duct 61 and a side hole 641. The side hole 641 is connected with the first duct 61 and faces the side of the first duct 61. The axial direction of the side hole 641 is provided with a threaded section 6411 and a sliding section 6412. The sliding section 6412 is adjacent to the first duct 61. The positioning member 62 may be slidably arranged on the sliding section 6412. The fixing screw 63 is configured to penetrate the threaded section 6411 and push the positioning member 62 to move into the first duct 61 to fix the fixing section 542.

The operator may keep the tension of the traction line 54 inconvenient after the sheath portion 4 is coupled with the connecting end 311. By tightening the fixing screw 63, the positioning member 62 may be pressed against the fixing section 542.

Further, as shown in Figs. 1 to 8, the main body 21 has a window 212. The window 212 passes through the side wall on one side of the first sliding track 211 and the length direction is parallel to the axial direction of the first sliding track 211. The head of the fixing screw 63 protrudes from the window 212. In the first position and the second position, the fixing screw 63 is moved to the edge position in the length direction of the window 212, respectively.

The window 212 of this embodiment may avoid the fixing screw 63. The dotted line IVa in Fig. 3 is the axis position of the fixing screw 63 at the first position and the second position. Through the cooperation of the window 212 and the fixing screw 63, the moving position of the fixing portion 6 in the first sliding track 211 may be further limited.

In some embodiments, as shown in Figs. 1 to 11, the base 64 and the fixing screw 63 are insulating parts. The positioning member 62 includes a plurality of connecting handles 621 and a sliding body 622 adapted to the sliding section 6412. A plurality of connecting handles 621 are protruded on the side where the sliding body 622 is away from the fixing screw 63. The fixing portion 6 also comprises a plurality of first elastic members 67 and a first electrical contact member 65. The first electrical contact member 65 is arranged on the base 64 and includes an electrical contact surface 651, and the electrical contact surface 651 is protruded in the first duct 61. The bottom of the side hole 641 is provided with a plurality of first positioning holes 6413, a plurality of first positioning holes 6413 are arranged on both sides of the first duct 61, and a plurality of first elastic members 67 are correspondingly arranged in a plurality of first positioning holes 6413.

At the same time, the fixing screw 63 is screwed into the threaded section 6411, the tail of the fixing screw 63 pushes the sliding body 622 close to the electrical contact surface 651 to clamp the fixing section 542, and the first electrical contact member 65 is electrically connected with the fixing section 542. At the same time, a plurality of connecting handles 621 are correspondingly threaded into a plurality of first positioning holes 6413 and are correspondingly connected with a plurality of first elastic members 67.

Specifically, each first elastic member 67 in this embodiment is a spring. One end of a spring butts with the bottom surface of a first positioning hole 6413, and the other end butts with the end face of a connecting handle 621.

Therefore, on the one hand, through the mutual cooperation of multiple connecting handles 621 and multiple first positioning holes 6413, this embodiment avoids the rotation of the sliding body 622 relative to the fixing section 542, causing damage to the traction line 54. On the other hand, when the fixing screw 63 is screwed out to the outside of the threaded section 6411, the first elastic members 67 may simultaneously jack up the positioning member 62 to facilitate the extraction of the traction line 54 from the fixing portion 6 and avoid the adhesion of the traction line 54 and the positioning member 62.

Specifically, the base 64 is provided with an accommodating groove 642, and the first electrical contact member 65 is arranged in the accommodating groove 642 and electrically connected with the power supply equipment through the second wire 27. The insulated base 64 and the fixing screw 63 may prevent the solidification current from conducting to the operator's hands.

In some embodiments, as shown in Fig. 7 and Fig. 10, the bottom of the accommodating groove 642 is provided with a gap 643. The second wire 27 may be connected with the first electrical contact member 65 through the gap 643. The bottom of the base 64 is also provided with a groove 644, the wrench is rotationally connected with the main body 21, and the top bulge of the wrench may extend into the groove 644 to drive the fixing portion 6 to move in a straight line.

Further, a trench 623 is arranged on the side of the sliding body 622 toward the traction line 54, and the depth and radian of the trench 623 are adapted to the top of the fixing section 542. When the sliding body 622 presses against the fixing section 542, the trench 623 may just press against the top surface of the fixing section 542. At the same time, the mutual cooperation of a plurality of connecting handles 621 and a plurality of first positioning holes 6413 may further ensure that the extension direction of the trench 623 is consistent with the length direction of the traction line 54.

In some embodiments, as shown in Figs. 1 to 8, the first sliding track 211 has a tail port 2111, and the tail port 2111 is located on the side of the first sliding track 211 away from the sheath portion 4. The driving component 2 also includes a sealing member 22, the sealing member 22 is an insulating piece and includes a limiting bulge, and the limiting bulge is provided with a third duct 221. The fixing section 542 is located in the first duct 61, and the traction section 543 extends to the outside of the main body 21 through the tail port 2111. The sealing member 22 plugs the tail port 2111, the third duct 221 and the first duct 61 extend along the same axis, and at least part of the traction section 543 is flexibly arranged through the third duct 221. At the second position, the limiting bulge is butted with the fixing portion 6.

The length of the traction section 543 in this embodiment may be configured to adapt to the size of the operator's hand, so as to facilitate the operator to carry out the pulling operation. At the same time, the limiting bulge may limit the displacement of the fixing portion 6, so as to prevent the distance between the first operating arm 51 and the second operating arm 52 from being too small and damaging human tissues. As shown in state Ie in Fig. 12, when the sheath portion 4 is mated with the connecting end 311, the operator may cut off at least part of the traction section 543. Then set the third duct 221 on it. Thus, in the process of pulling the first operating arm 51 by the traction line 54, the traction line 54 may realize the simultaneous telescopic movement in the first duct 61, the second duct 312 and the third duct 221, so as to avoid the interference between the end of the traction section 543 and the blocking part 22, thereby affecting the pulling action of the traction portion 53.

In some embodiments, as shown in Figs. 1 to 8, the connecting portion 3 includes a first connector 32, and the first connector 32 is arranged at the end of the rod member 31 far from the connecting end 311. The main body 21 includes a containing portion 23 and a second connector 24. The containing portion 23 is provided with a first sliding track 211, the second connector 24 is protruded on one side of the containing portion 23 and is provided with a fourth duct 241, and the traction line 54 may operably pass through the fourth duct 241 and the first sliding track 211. The driving component 2 also includes the first wire 25. The first connector 32 is connected with the second connector 24, the fourth duct 241 and the first duct 61 extend along the same axis, and one end of the first wire 25 is electrically connected with the first connector 32, and the other end extends to the outside of the main body 21. In this embodiment, one end of the first wire 25 is electrically connected with the second operating arm 52, and the other end is electrically connected with the power supply equipment to feed the second operating arm 52.

Further, the driving component 2 also includes a second electrical contact member 26. The second electrical contact member 26 is provided with a central through hole 261 sleeved on the traction line 54. The second connector 24 provides a fifth duct 242, which is connected with the first sliding track 211 and parallel to the fourth duct 241. The first connector 32 and the second connector 24 are arranged in pairs and are connected. The second electrical contact member 26 is butted between the end face of the rod member 31 and the second connector 24, and the central through hole 261 is aligned with the first duct 61 and the fourth duct 241. The first wire 25 is connected with the second electrical contact member 26 through the first sliding track 211 and the fifth duct 242. In this embodiment, the contact area with the rod member 31 is increased through the second connector 24 to facilitate the current conduction in the first wire 25.

Specifically, the above first duct 61, second duct 312, third duct 221 and fourth duct 241 extend along the same axis, that is, first duct 61, second duct 312, third duct 221 and fourth duct 241 are aligned. In addition, the inner diameters of first duct 61, second duct 312, third duct 221 and fourth duct 241 are all larger than the outer diameter of the traction line 54, so as to ensure that the traction line 54 may be flexibly and simultaneously threaded in each hole.

Optionally, the first electrical contact member 65 and the second electrical contact member 26 are both elastic contact members. That is, the electrical contact surface 651 has an elastic deformation amount toward the outside of the first duct 61. The second electrical contact member 26 is an elastic sheet, and in the thickness direction, both sides of the elastic sheet have elastic deformation toward the middle area. Thus, the first electrical contact member 65 and the second electrical contact member 26 are guaranteed to realize stable ground electrical connection.

In some embodiments, as shown in Figs. 1 to 8, the traction portion 53 also includes a moving head 531. The moving head 531 is connected with the tail of the connecting section 541 and the first operating arm 51. The sheath portion 4 comprises a second elastic member 41, a second sliding track 42, a stop ring surface 43 and a first containing hole 44. The first containing hole 44 is a through hole, the stop ring surface 43 is located between the second sliding track 42 and the first containing hole 44, the moving head 531 is slidably arranged in the second sliding track 42, and the second elastic member 41 is butted between the moving head 531 and the stop ring surface 43. When the traction portion 53 is in a free state, the second elastic member 41 pushes the first operating arm 51 away from the second operating arm 52. In the splicing state, at least part of the connecting end 311 is inserted into the first containing hole 44.

The moving head 531 in this embodiment may play a guiding role by cooperating with the second sliding track 42. The second elastic member 41 may ensure that the first operating arm 51 and the second operating arm 52 are in an open state when the fixing portion 6 does not pull the moving head 531.

In some embodiments, as shown in Figs. 1 to 8, the connecting end 311 includes a contact ring surface 3111 and a puncture head 3112, and the puncture head 3112 is projected on the central area of the contact ring surface 3111. The sheath portion 4 also comprises an elastic sleeve 45. The elastic sleeve 45 is arranged on the second sliding track 42, the second elastic member 41 is butted on the stop ring surface 43 through the elastic sleeve 45, the elastic sleeve 45 is provided with a tapered hole 451, the small diameter end of the tapered hole 451 is far away from the stop ring surface 43, and at least the inner diameter of some tapered hole 451 is smaller than the outer diameter of the puncture head 3112. In the above splicing state, the puncture head 3112 is simultaneously penetrated into the first containing hole 44 and the tapered hole 451 until it butting the ring surface 3111 and the sheath portion 4.

This embodiment limits the relative position relationship between the sheath portion 4 and the driving component 2 by matching the contact ring surface 3111 and the sheath portion 4. When the puncture head 3112 is inserted into the sheath portion 4, the elastic sleeve 45 may wrap the puncture head 3112 to prevent the connecting end 311 from easily coming out of the sheath portion 4. For example, the operator holds the main body 21 with his left hand and pulls the traction section 543 with his right hand. When the puncture head 3112 is inserted into the elastic sleeve 45, the operator may loosen his right hand and tighten the fixing screw 63 with his right hand. Simplified the operational difficulty for operators. At the same time, it may also avoid the easy rotation of the operating components along the circumference of the rod during the use of intracavitary surgical instruments.

Optionally, the connecting end 311 includes a plug connector, and the end of the plug connector may be a pointed end or a flat end. When configuring the end of the connector as a flat head, the operator may insert the connector through the puncture needle or directly into the first incision, thereby allowing some of the rod 31 to be inserted into the cavity. When the end of the plug connector is configured as a tip (that is, the plug connector forms a puncture head 3112), it is convenient for the operator to insert some rod pieces 31 into the cavity.

Specifically, when the operator disassembles the intracavitary surgical instrument, the operator may unscrew the fixing screw 63 to the outside of the threaded section 6411, so that the positioning member 62 is separated from the traction line 54, but the puncture head 3112 is still in the elastic sleeve 45, and the friction between the puncture head 3112 and the elastic sleeve 45 may keep them connected.

In this form, the operator may pull out the rod member 31 directly from the first wound or from the puncture needle with one hand. When the rod member 31 is pulled out from the puncture needle, the operator may hold one end of the puncture needle with the other hand, so that the operating component or the electrocoagulation component is butted with the other end of the puncture needle, so that the operating component or the electrocoagulation component is separated from the rod member 31. When the rod member 31 is pulled out from the first wound, the operator may press it near the first wound with the other hand to make the operating component or the electrocoagulation component touch the skin, so as to separate the operating component or the electrocoagulation component from the rod member 31.

Therefore, the cooperation between the puncture head 3112 and the elastic sleeve 45 may not only ensure the stability of the connection of the intracavitary surgical instrument during assembly, but also further simplify the operation steps when the operating component or the electrocoagulation component is separated from the rod member 31.

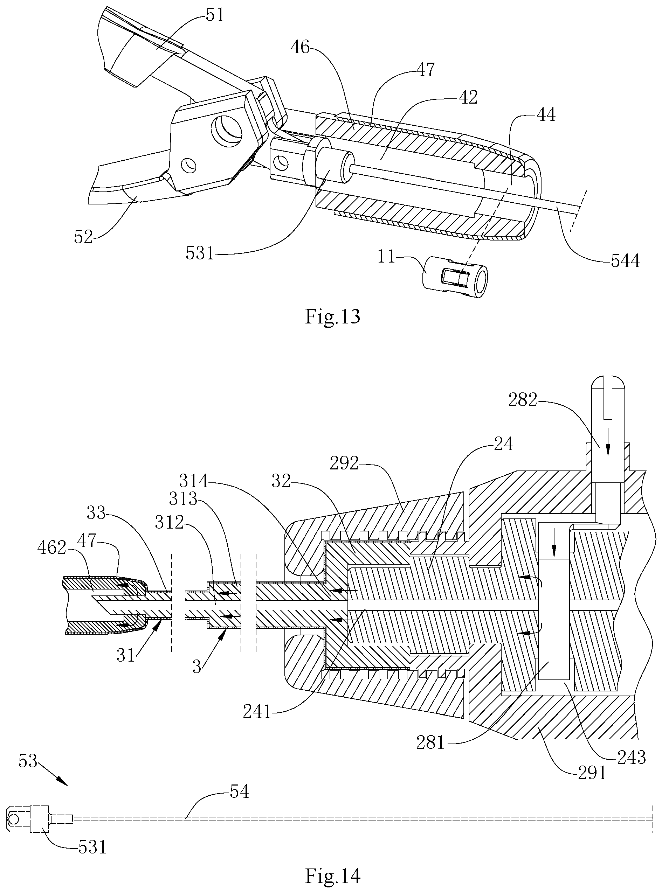

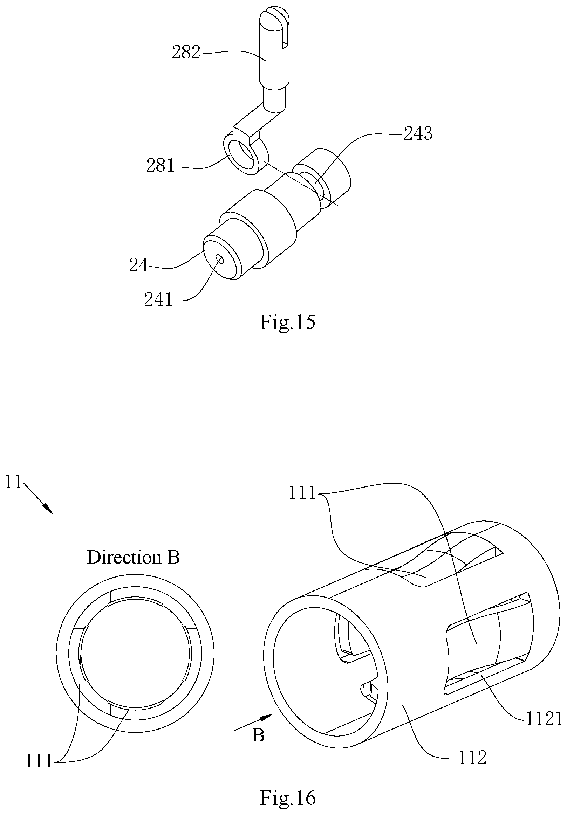

In some embodiments, as shown in Fig. 13 and Fig. 16, the operating component also includes a connecting sleeve 11. The connecting sleeve 11 includes a plurality of positioning elastic sheets 111, and the plurality of positioning elastic sheets 111 have an elastic bending amount toward the outside of the connecting sleeve 11. That is, after the connector is inserted into the connecting sleeve 11, the connector will push the plurality of positioning elastic sheets 111 to make the positioning elastic sheets 111 move outward.

The connecting sleeve 11 is arranged on the first containing hole 44 so that the connecting sleeve 11 is fixedly connected with the sheath portion 4. The connector is inserted into the connecting sleeve 11, and the plurality of positioning elastic sheets 111 are butted against the lateral side of the connector.

In this embodiment, the relative position relationship between the sheath portion 4 and the driving component 2 may be limited by using the cooperation of the plurality of positioning elastic sheets 111 with the socket. In the process of inserting the connector into the sheath portion 4, the connecting sleeve 11 may wrap the puncture head 3112 to prevent the connecting sleeve 11 from easily falling out of the sheath portion 4.

Further, as shown in Fig. 16, the connecting sleeve 11 in the figure includes a fixed cylinder 112, and the fixed cylinder 112 is provided with avoidance holes 1121. The positioning elastic sheets 111 are arranged at the avoidance holes 1121, and the plurality of positioning elastic sheets 111 are uniformly distributed along the circumference of the fixed cylinder 112. Each positioning elastic sheet 111 bends towards the inside of the fixed cylinder 112 in a free state. The connecting sleeve 11 is clamped in the first containing hole 44 through the fixed cylinder 112. At the same time, each positioning elastic sheet 111 has a certain position end in contact with the socket joint, and in the axial section direction of the fixed cylinder 112, the first locating end bends to the outside of the fixed cylinder 112. The plurality of first positioning ends in this embodiment may be adapted to the side shape of the plug connector to ensure that the connecting sleeve 11 has sufficient contact area with the plug connector. At the same time, the friction between the plurality of positioning elastic sheets 111 and the socket joint may also prevent the operating component from easily rotating along the circumference of the rod member piece 31 when the intracavitary surgical instrument is in use.

In some embodiments, as shown in Figs. 1 to 8, the rod member 31 is a conductive part and includes a transition section 313 and a opposite end 314. The transition section 313 is located between the connecting end 311 and the opposite end 314. The connecting portion 3 also includes a first insulation coating layer 33, the first insulation coating layer 33 is coated on the transition section 313, and the connecting end 311 is exposed outside the first insulation coating layer 33. The sheath portion 4 also includes the conductive body 46 and the second insulation coating layer 47 coated on the outer part area of the conductive body 46, the second operating arm 52 is arranged on the conductive body 46, and the conductive body 46 has a match end surface 461. The match end surface 461 is exposed outside the second insulation coating layer 47 and butts with the contact ring surface 3111. In this embodiment, the electrical connection of the second electrical path may be realized through the contact between the match end surface 461 and the contact ring surface 3111.

Specifically, as shown in Figs. 5, 8 and 9, one edge of the second insulation coating layer 47 extends to the edge of the match end surface 461. The edge of one side of the first insulation coating layer 33 extends to the contact ring surface 3111. When the sheath portion 4 is spliced with the rod member 31, the first insulation coating layer 33 and the second insulation coating layer 47 may also be spliced with each other, so as to reduce or even avoid the short connection between the connection position of the conductive body 46 and the rod member 31 exposed in the human body.

Specifically, as shown in Fig. 8, the first connector 32 includes an extended edge around the rod member 31. The protruding edge sleeve is arranged at the end of the second connector 24 to ensure the radial position accuracy. At the same time, the axial positioning of the first connector 32 and the second connector 24 is realized by screwing the first connector and the second connector arranged outside the second connector 24 and the first connector 32. The first insulation coating layer 33 is also coated on the outside of the protruding edge.

In some embodiments, as shown in Figs. 1 to 8, the sheath portion 4 also includes a conductive body 46 and an insulation lining 48. The conductive body 46 has a second containing hole 462, a retaining ring surface 43 and a first containing hole 44, and the second containing hole 462 has an open end 4621 far from the first containing hole 44. The insulation lining 48 includes outer flanges 481 and a cylinder body 482, and the inner wall of the cylinder body 482 forms a second sliding track 42. The cylinder body 482 is arranged in the second containing hole 462, and the outer flange 481 is clamped at the open end 4621.

Optionally, in this embodiment, the insulation lining 48 is made of ceramic material, which may play a good insulating role. At the same time, the surface of the ceramic material is smooth, which may also play a good role in guiding the movement of the moving head 531. Moreover, the valgus flanges 481 may also play a limiting role to ensure the installation accuracy between the conductive body 46 and the insulation lining 48.

Further, the moving portion 5 also includes an insulation spacer block 56, and the insulation spacer block 56 is arranged between the first operating arm 51 and the second operating arm 52. The number of outward turning edges 481 is two and their extension directions are opposite. The conductive body 46 comprises two supporting arms 464, and two supporting arms 464 are projected on the open end 4621 and staggered with two outer flanges 481. The insulation spacer block 56 has a first hinge hole B1 and a second hinge hole B2, the second operating arm 52 has a third hinge hole B3 and a fourth hinge hole B4, the first operating arm 51 has a fifth hinge hole B5 and a sixth hinge hole B6, the supporting arm 464 has a seventh hinge hole B7, and the moving head 531 has an eighth hinge hole B8. The first hinge hole B1 and the third hinge hole B3 are simultaneously hinged with the seventh hinge hole B7, the second hinge hole B2 and the fourth hinge hole B4 It is hinged with the fifth hinge hole B5, the eighth hinge hole B8 and the sixth hinge hole B6, and the two valgus edges 481 correspond to the first operating arm 51 and the second operating arm 52 respectively.

In this embodiment, the two valgus edges 481 may prevent the opening angle of the first operating arm 51 and the second operating arm 52 from being too large to collide with the conductive body 46, and may also prevent the short circuit between the first operating arm 51 and the conductive body 46.

Optionally, as shown in Fig. 4, the electrocoagulation component 1 further comprises a rotating shaft. The hole diameters of the second hinge hole B2, the fourth hinge hole B4 and the fifth hinge hole B5 in the figure are larger than those of other hinge holes. When the rotating shaft passes through the second hinge hole B2, the fourth hinge hole B4 and the fifth hinge hole B5, the linear motion of the moving head 531 may be converted into the relative rotation of the first operating arm 51 and the second operating arm 52 through a larger radial clearance to avoid interference caused by small gaps.

In some embodiments, as shown in Figs. 1 to 8, the traction line 54 also includes a conductive core 544 and a third insulation coating 545. The third insulating coating 545 is coated on the corresponding area between the conductive core 544 and the rod member 31, and the corresponding area between the conductive core 544 and the fixing portion 6 is exposed outside the third insulating coating 545. The third insulating coating 545 may also be coated on the lateral side of the moving head 531.

In this embodiment, the third insulation coating 545 may avoid the short connection between the conductive core 544 and the rod member 31, and the fixing section 542 and the traction section 543 do not cover the third insulation coating 545, so as to facilitate the electrical connection with the power supply equipment.

Fig. 13 is a schematic sectional view of the electrocoagulation surgical instrument in this embodiment. Fig. 14 is a partial explosion diagram of the driving component 2 in this embodiment. Fig. 14 further shows the corresponding relationship between each area on the traction portion 53 and the driving component 2 and the sheath portion 4. Fig. 15 is an exploded schematic diagram of the conductive ring 281, the conductive arm 282 and the second connector 24 of this embodiment.

In some embodiments, as shown in Figs. 1 to 2, the electrocoagulation surgical instrument in this embodiment includes an electrocoagulation component 1 and a driving component 2.

Further referring to Fig. 13 and Fig. 14, the driving component 2 includes a connecting portion 3. The connecting portion 3 comprises a hollow rod member 31, and the rod member 31 comprises a connecting end 311. Further referring to Fig. 6 and Fig. 15, the electrocoagulation component 1 includes a sheath portion 4, a traction portion 53, and a moving portion 5 arranged on the sheath portion 4. The moving portion 5 includes a first operating arm 51 and a second operating arm 52.

The traction portion 53 is configured such that one end is connected with the moving portion 5, the other end passes through the rod member 31 in an operable manner, and the sheath portion 4 is pulled to move towards the rod member 31 until the connecting end 311 is detachably connected with the sheath portion 4. The traction portion 53 is used to drive the first operating arm 51 close to the second operating arm 52. The moving portion 5 is configured to make the first operating arm 51 and the second operating arm 52 conduct with the rod member 31 through the sheath portion 4, and/or the first operating arm 51 and the second operating arm 52 conduct with the traction portion 53.

Specifically, the rod member 31 and the traction portion 53 in this embodiment are configured as conductive parts. The power supply device may supply power to the first operating arm 51 and the second operating arm 52 through the rod 31 (the arrow in Fig. 13 shows the current flow direction), so that an electric coagulation current is generated between the first operating arm 51 and the second operating arm 52. The power supply device may also supply power to the first operating arm 51 and the second operating arm 52 through the traction portion 53.

The electrocoagulation surgical instrument in this embodiment may be used as a monopolar electrocoagulation device. The operating table is provided with a ground plate, and the first operating arm 51 and the second operating arm 52 form a path between the biological tissue between them and the ground plate, so that the electrocoagulation current applied to the first operating arm 51 and the second operating arm 52 may reduce the bleeding of the patient's wounds. The first operating arm 51 and the second operating arm 52 in this embodiment may be configured in the form of a pliers component, a scissors component, a forceps component, or the like. Technicians in this field may choose based on the actual operation of the surgery.

To sum up, the electrocoagulation surgical instrument of this embodiment electrically connects the traction portion 53 or the rod member 31 with the first operating arm 51 and the second operating arm 52 to apply coagulation current to biological tissues. Therefore, on the one hand, the electrocoagulation surgical instrument is configured as detachable and spliced electrocoagulation component 1 and driving component 2, which makes full use of multiple wounds of endoscopic surgery, so that the electrocoagulation component 1 and rod member 31 may be placed into the cavity from different wounds, reducing the corresponding wound size of rod member 31, and improving the recovery speed of patients after surgery. On the other hand, during the assembly process of the electrocoagulation component 1 and the driving component 2, the traction portion 53 may pull the electrocoagulation component 1 and the driving component to move in opposite directions, so as to facilitate the assembly of the two. At the same time, the traction portion 53 may further make the first operating arm 51 and the second operating arm 52 close to each other through the pulling action, so as to realize the electrocoagulation of biological tissues by the first operating arm 51 and the second operating arm 52, and simplify the operation of surgery.

In some embodiments, as shown in Figs. 1 to 12, the electrocoagulation surgical instrument in the above embodiments may be used as an intracavitary surgical instrument. The intracavitary surgical instrument comprises an operating component and a driving component 2. The driving component 2 comprises a connecting portion 3, the connecting portion 3 comprises a hollow rod member 31, the rod member 31 comprises a connecting end 311, the connecting end 311 comprises a contact ring surface 3111 and an insertion head, and the insertion head is projected on the contact ring surface 3111. The operating component comprises a sheath portion 4 and a moving portion 5, the sheath portion 4 has a match end surface 461 and a first containing hole 44, the first containing hole 44 is a through hole, the moving portion 5 comprises a traction portion 53, a first operating arm 51 and a second operating arm 52, and the second operating arm 52 is arranged on the sheath portion 4.

The traction portion 53 is configured such that one end is connected with the first operating arm 51, the other end is operable through the rod member 31, and the sheath portion 4 is pulled to move towards the rod member 31 until the socket is detachably arranged through the first containing hole 44, and the contact ring surface 3111 is butted with the match end surface 461. Meanwhile, the traction portion 53 is used to drive the first operating arm 51 close to the second operating arm 52.

Therefore, multiple wounds of endoscopic surgery are fully utilized, so that the operating component and rod member 31 may be placed into the cavity from wounds at different positions, reducing the corresponding wound size of rod member 31, and improving the recovery speed of patients after surgery.

In some embodiments, as shown in Figs. 1 to 8, the traction portion 53 is configured to connect one end with the first operating arm 51, the second operating arm 52 is arranged on the sheath portion 4, and the first operating arm 51 is rotationally connected with the second operating arm 52 and electrically contacted. The voltages on the first operating arm 51 and the second operating arm 52 in this embodiment are the same.

The traction portion 53 includes a traction line 54, and the traction line 54 includes a connecting section 541 and a fixing section 542. The connecting section 541 is close to the first operating arm 51. The driving component 2 also includes a fixing portion 6, which is arranged on the side of the connecting part 3 away from the electrocoagulation component 1 and is provided with a first duct 61. The fixing section 542 extends through the rod member 31 into the first duct 61 and is fixedly connected with the fixing portion 6, which is operably close to or far from the connecting portion 3 to drive the first operating arm 51 to move relative to the second operating arm 52.

In this embodiment, the traction line 54 is flexible, so that the electrocoagulation component 1 may be placed into the cavity through the first wound. At the same time, in the first stage, the second stage and the third stage, the traction line 54 may provide the pulling force required for the proximity of the electrocoagulation component 1 and the driving component 2, and the proximity of the first operating arm 51 and the second operating arm 52. At the same time, it may also be used as a wire to guide the solidification current.

In some embodiments, as shown in Figs. 1 to 8, the first duct 61 is a through hole. The traction line 54 also includes a traction section 543. The driving component 2 also comprises a main body 21, and one end of the connecting portion 3 far from the connecting end 311 is connected with the main body 21.

The electrocoagulation surgical instruments are in an assembled state. In the splicing state, the traction section 543 extends from the end of the first duct 61 far from the connecting section 541, and pulls the sheath portion 4 to splice with the connecting end 311, and the fixing section 542 is located in the first duct 61.

The traction section 543 in this embodiment is used for the operator to pull the electrocoagulation component 1, so that the electrocoagulation component 1 and the driving component are mutually matched. When the sheath portion 4 and the connecting end 311 are spliced together, ensure that the fixing section 542 is just located in the first duct 61. So that the operator may fix the fixing section 542 located in the first duct 61 through the fixing section 6. On the other hand, the coupling soft tube 7 may be sleeved on the traction section 543, so that the coupling soft tube 7 may lead the traction line 54 out to the outside of the human body through the first wound.

In some embodiments, as shown in Fig. 14, the connecting portion 3 includes a first connector 32, and the first connector 32 is arranged at the end of the rod member 31 far from the connecting end 311. The main body 21 includes a containing portion 23 and a second connector 24.

Further referring to Fig. 7, the containing portion 23 is provided with a first sliding track 211, and the fixing portion 6 is slidably arranged on the first sliding track 211. The fixing portion 6 has a first position and a second position in the first sliding track 211. In the first position, the fixing portion 6 is located at one end of the first sliding track 211 near the sheath part 4, and the first duct 61 and the second duct 312 extend along the same axis. In the second position, the fixing portion 6 is located at the end of the first sliding track 211 away from the sheath part 4, and the first operating arm 51 is close to the second operating arm 52. The second connector 24 is a conductive part and is provided with a fourth duct 241, and the traction line 54 may operably pass through the fourth duct 241 and the first sliding track 211. The first connector 32 is fixedly connected with the second connector 24, and the second connector 24 is conductive to the sheath portion 4 through the rod piece 31.

Optionally, the traction line 54 in this embodiment is a conductive core 544. The conductive core 544 comprises a plurality of strands of metal wires. Multiple strands of metal wires are spirally coiled together to increase the mechanical strength of the traction portion. At the same time, the resistance of the conductive core 544 may be reduced.

In this embodiment, the second connector is used to feed the rod member, so that the current conduction on the rod member and the second connector is more stable. At the same time, the current may also be transmitted to the conductive core 544 located in the second duct 312 and fourth duct 241, so that the conductive core 544 and the rod member conduct current together.

In some embodiments, as shown in Figs. 14 to 15, the second connector 24 is provided with a positioning groove 243, and the positioning groove 243 is concave from the outside of the second connector 24 to the fourth duct 241.

The driving component 2 also includes a housing portion 291 and a conductive ring 281. The conductive ring 281 is arranged in the positioning groove 243 and electrically connected with the second connector 24. At least part of the second connector 24 is arranged on the inner side of the housing portion 291, and the housing portion 291 is an insulating part.

Specifically, as shown in Fig. 14, the driving component 2 also includes a locking nut 292. The locking nut 292 is an insulating part and is threaded with the housing portion 291, so that the first connector 32 and the second connector 24 are fixedly connected together. At the same time, the first connector 32 and the second connector 24 are located on the inside of the locking nut 292 and the housing portion 291 to avoid electric shock to the operator. The conductive ring 281 in this embodiment is clamped in the positioning groove 243 to ensure the stable connection between the conductive ring 281 and the second connector 24.

In some embodiments, as shown in Figs. 14 to 15, the driving component 2 also includes a conductive arm 282. One end of the conductive arm 282 is connected with the conductive ring 281, and the other end extends to the outside of the housing portion 291. In this embodiment, one end of the conductive arm extends to the top of the driving component 2, and the conductive arm may be connected with the feed cable of the power supply equipment.

In some embodiments, as shown in Figs. 1 to 8 and 13, the traction portion 53 also includes a moving head 531, which is connected with the tail of the connecting section 541 and the first operating arm 51. The sheath portion 4 comprises a second elastic member 41, a second sliding track 42, a stop ring surface 43 and a first containing hole 44. The first containing hole 44 is a through hole, the stop ring surface 43 is located between the second sliding track 42 and the first containing hole 44, the moving head 531 is slidably arranged in the second sliding track 42, and the second elastic member 41 is butted between the moving head 531 and the stop ring surface 43.

When the traction portion 53 is in a free state, the second elastic member 41 pushes the first operating arm 51 away from the second operating arm 52. In the splicing state, at least part of the connecting end 311 is inserted into the first containing hole 44.

The moving head 531 in this embodiment may play a guiding role by cooperating with the second sliding track 42. The second elastic member 41 may ensure that the first operating arm 51 and the second operating arm 52 are in an open state when the fixing portion 6 does not pull the moving head 531. The moving head 531 may directly contact the conductive body 46, making the current transfer between them, further ensuring that the voltage of the first operating arm and the second operating arm is the same.

In some embodiments, as shown in Figs. 1 to 8, the connecting end 311 includes an contact ring surface 3111 and an insertion head, and the insertion head is projected on the central area of the contact ring surface 3111. The sheath portion 4 also comprises an elastic sleeve 45. The elastic sleeve 45 is arranged on the second sliding track 42, the second elastic member 41 is butted on the stop ring surface 43 through the elastic sleeve 45, the elastic sleeve 45 is provided with a tapered hole 451, the small diameter end of the tapered hole 451 is far away from the stop ring surface 43, and at least part of the inner diameter of the tapered hole 451 is smaller than the outer diameter of the socket. In the splicing state, the socket joint is simultaneously penetrated into the first containing hole 44 and the tapered hole 451 until the contact ring surface 3111 butts against the sheath portion 4.

This embodiment limits the relative position relationship between the sheath portion 4 and the driving component 2 by matching the contact ring surface 3111 and the sheath portion 4. During the insertion of the connector into the sheath portion 4, the elastic sleeve 45 may wrap the connector to prevent the connecting end 311 from easily coming out of the sheath portion 4.

In some embodiments, as shown in Figs. 1 to 8 and Figs. 13 to 14, the rod member 31 is a conductive part and includes a transition section 313 and an opposite end 314, the transition section 313 is located between the connecting end 311 and the opposite end 314, and the first connector 32 is arranged at the opposite end 314. The connecting portion 3 also includes a first insulation coating layer 33, the first insulation coating layer 33 is coated on the transition section 313, and the connecting end 311 is exposed outside the first insulation coating layer 33. The sheath portion 4 also includes the conductive body 46 and the second insulation coating layer 47 coated on the outer part area of the conductive body 46, the second operating arm 52 is arranged on the conductive body 46, and the conductive body 46 has a match end surface 461. The match end surface 461 is exposed outside the second insulation coating layer 47 and butts with the contact ring surface 3111. In this embodiment, the electrical connection between the rod member and the sheath may be realized by butting the match end surface 461 and the contact ring surface 3111.

The above description is only a preferred embodiment of the present disclosure and is not intended to limit the present disclosure. For those skilled in the art, the present disclosure may have various modifications and variations. Any modifications, equivalent substitutions, improvements, etc. made within the spirit and principles of the present disclosure may be included within the scope of protection of the present disclosure.

## Claims

1. An intracavitary surgical instrument, comprising:
a driving component (2), comprising a connecting portion (3), the connecting portion (3) comprising a hollow rod member (31), the rod member (31) comprising a connecting end (311), the connecting end (311) comprising a contact ring surface (3111) and an insertion head, the insertion head protruding from the contact ring surface (3111); and
an operating component, comprising a sheath portion (4) and a moving portion (5), the sheath portion (4) having a match end surface (461) and a first containing hole (44), the first containing hole (44) being a through hole, the moving portion (5) comprising a traction portion (53), a first operating arm (51), and a second operating arm (52), the second operating arm (52) being disposed on the sheath portion (4);
wherein one end of the traction portion (53) is connected to the first operating arm (51), and the other end of the traction portion (53) is operably passed through the rod member (31) and pulls the sheath portion (4) to move toward the rod member (31) until the insertion head is detachably inserted through the first containing hole (44), and the contact ring surface (3111) abuts the match end surface (461), the traction portion (53) being configured to drive the first operating arm (51) to approach the second operating arm (52).

2. The intracavitary surgical instrument according to claim 1, wherein the traction portion (53) comprises a traction line (54), the traction line (54) comprising a connecting section (541) and a fixing section (542), the connecting section (541) being proximate to the first operating arm (51);
the driving component (2) further comprises a fixing portion (6), the fixing portion (6) being disposed on a side of the connecting portion (3) away from the operating component and provided with a first duct (61);
the fixing section (542) passes through the rod member (31) into the first duct (61) and is fixedly connected to the fixing portion (6), the fixing portion (6) being operably moved toward or away from the connecting portion (3) to drive the first operating arm (51) to move relative to the second operating arm (52).

3. The intracavitary surgical instrument according to claim 2, wherein the first duct (61) is a through hole;
the traction line (54) further comprises a traction section (543);
the driving component (2) further comprises a main body (21);
an end of the connecting portion (3) away from the connecting end (311) is connected to the main body (21);
the intracavitary surgical instrument has an assembled state, in the assembled state, the traction section (543) extends from an end of the first duct (61) away from the connecting section (541) and pulls the sheath portion (4) to assemble with the connecting end (311), and the fixing section (542) is located within the first duct (61).

4. The intracavitary surgical instrument according to claim 3, wherein the rod member (31) is provided with a second duct (312), the second duct (312) is a through hole;
the main body (21) is provided with a first sliding track (211), the fixing portion (6) is slidably disposed on the first sliding track (211), the fixing portion (6) has a first position and a second position within the first sliding track (211), in the first position, the fixing portion (6) is located at an end of the first sliding track (211) proximate to the sheath portion (4) and the first duct (61) and the second duct (312) extend along the same axis, in the second position, the fixing portion (6) is located at an end of the first sliding track (211) away from the sheath portion (4), the first operating arm (51) approaches the second operating arm (52) and maintains a predetermined distance.

5. The intracavitary surgical instrument according to claim 4, wherein an axial direction of the first sliding track (211) is parallel to the first duct (61) and the second duct (312).

6. The intracavitary surgical instrument according to claim 4, wherein a port on a side of the first duct (61) opposite to the second duct (312) has a predetermined distance;
the fixing portion (6) is provided with a guiding tapered surface (66), a small-diameter end of the guiding tapered surface (66) is connected to a port of the first duct (61) proximate to the second duct (312).

7. The intracavitary surgical instrument according to claim 6, wherein the traction line (54) is flexible;
the main body (21) has a fourth duct (241) and a containing cavity, the traction line (54) is operably passed through the fourth duct (241) and the first sliding track (211), the containing cavity is located between the fourth duct (241) and the first sliding track (211), the fixing portion (6) moves toward the sheath portion (4), and a region of the containing cavity between the fourth duct (241) and the fixing portion (6) forms an avoidance space;
the traction line (54) extends into the containing cavity, an end of the traction line (54) is placed on the guiding tapered surface (66) and slides from the guiding tapered surface (66) into the first duct (61).

8. The intracavitary surgical instrument according to claim 4, wherein the fixing portion (6) further comprises:
a positioning member (62);
a fixing screw (63); and
a base (64), provided with the first duct (61) and a side hole (641), the side hole (641) communicates with the first duct (61) and faces a lateral direction of the first duct (61), the side hole (641) has a threaded section (6411) and a sliding section (6412) in an axial direction, the sliding section (6412) is adjacent to the first duct (61), the positioning member (62) is slidably disposed in the sliding section (6412);
the fixing screw (63) is configured to pass through the threaded section (6411) and push the positioning member (62) to move into the first duct (61) to fix the fixing section (542).

9. The intracavitary surgical instrument according to claim 8, wherein the main body (21) is provided with a window (212), the window (212) penetrates a side wall of one side of the first sliding track (211) and has a length direction parallel to the axial direction of the first sliding track (211);
a head of the fixing screw (63) extends out from the window (212), and in the first position and the second position, the fixing screw (63) is moved to edge positions along the length direction of the window (212).

10. The intracavitary surgical instrument according to claim 8, wherein the positioning member (62) comprises a plurality of connecting handles (621) and a sliding body (622) adapted to the sliding section (6412), the plurality of connecting handles (621) protrude from a side of the sliding body (622) away from the fixing screw (63);
the fixing portion (6) further comprises:
a plurality of first elastic members (67); and
a plurality of first positioning holes (6413) provided at a bottom of the side hole (641), the plurality of first positioning holes (6413) are arranged on both sides of the first duct (61), the plurality of first elastic members (67) are respectively disposed within the plurality of first positioning holes (6413);
the fixing screw (63) is screwed into the threaded section (6411), a tail of the fixing screw (63) pushes the sliding body (622) toward the first duct (61) to clamp the fixing section (542), and the plurality of connecting handles (621) are respectively inserted into the plurality of first positioning holes (6413) and abut the plurality of first elastic members (67).

11. The intracavitary surgical instrument according to claim 10, wherein when the fixing screw (63) is unscrewed from the threaded section (6411), the first elastic members (67) lift the positioning member (62).

12. The intracavitary surgical instrument according to claim 4, wherein the first sliding track (211) has a tail port (2111), the tail port (2111) is located on a side of the first sliding track (211) away from the sheath portion (4);
the driving component (2) further comprises a sealing member (22), the sealing member (22) comprising a limit protrusion, the limit protrusion being provided with a third duct (221);
the fixing section (542) is located within the first duct (61), the traction section (543) extends out through the tail port (2111) to an exterior of the main body (21);
the sealing member (22) seals the tail port (2111), the third duct (221) and the first duct (61) extend along the same axis, and at least part of the traction section (543) is retractably passed through the third duct (221);
in the second position, the limit protrusion abuts the fixing portion (6).

13. The intracavitary surgical instrument according to claim 2, further comprising:
a puncture needle; and
a coupling soft tube (7), being a hollow structure and comprising a head portion, a tail portion, and an intermediate region between the head portion and the tail portion;
the coupling soft tube (7) passes through the puncture needle, and the puncture needle is located between the intermediate region and the tail portion, the head portion is configured to couple with the traction line (54), and the tail portion is configured to drive the traction line (54) through the puncture needle.

14. The intracavitary surgical instrument according to claim 3, wherein the traction portion (53) further comprises a moving head (531), the moving head (531) being connected to the connecting section (541) and a tail end of the first operating arm (51);
the sheath portion (4) comprises a second elastic member (41), a second sliding track (42), and a stop ring surface (43), the stop ring surface (43) is located between the second sliding track (42) and the first containing hole (44), the moving head (531) is slidably disposed within the second sliding track (42), and the second elastic member (41) abuts between the moving head (531) and the stop ring surface (43);
in a free state of the traction portion (53), the second elastic member (41) pushes the first operating arm (51) away from the second operating arm (52);
in the assembled state, at least part of the connecting end (311) is inserted into the first containing hole (44).

15. The intracavitary surgical instrument according to claim 14, wherein the insertion head is a puncture head (3112), the puncture head (3112) protruding from a central region of the contact ring surface (3111);
the sheath portion (4) further comprises:
an elastic sleeve (45), disposed on the second sliding track (42), the second elastic member (41) abuts against the stop ring surface (43) via the elastic sleeve (45), the elastic sleeve (45) is provided with a tapered hole (451), a small-diameter end of the tapered hole (451) is away from the stop ring surface (43), and an inner diameter of at least part of the tapered hole (451) is smaller than an outer diameter of the insertion head;
in the assembled state, the puncture head (3112) is simultaneously inserted through the first containing hole (44) and the tapered hole (451) until the contact ring surface (3111) abuts the match end surface (461).

16. The intracavitary surgical instrument according to claim 1, wherein the operating component further comprises a connecting sleeve (11), the connecting sleeve (11) comprises a plurality of positioning elastic sheets (111), the plurality of positioning elastic sheets (111) having elastic bending amounts toward an exterior of the connecting sleeve (11);
the connecting sleeve (11) is disposed in the first containing hole (44), the insertion head is inserted into the connecting sleeve (11), and the plurality of positioning elastic sheets (111) abut against a lateral side of the insertion head.

17. The intracavitary surgical instrument according to claim 1, wherein the rod member (31) comprises a first extension section and a second extension section in a length direction, a diameter of the first extension section is less than 3 millimeters, and the diameter of the first extension section is smaller than a diameter of the second extension section.

18. An electrocoagulation surgical instrument, comprising:
a driving component (2), comprising a connecting portion (3), the connecting portion (3) comprising a hollow rod member (31), the rod member (31) comprising a connecting end (311); and
an electrocoagulation component (1), comprising a sheath portion (4) and a moving portion (5), the moving portion (5) comprising a traction portion (53), a first operating arm (51), and a second operating arm (52), the second operating arm (52) being disposed on the sheath portion (4);
wherein the traction portion (53) is configured such that one end is connected to the first operating arm (51) and the traction portion (53) and the first operating arm (51) form a first electrical pathway, the other end is operably passed through the rod member (31) and pulls the sheath portion (4) to move toward the rod member (31) until the connecting end (311) is detachably connected to the sheath portion (4), so that the rod member (31), the sheath portion (4), and the second operating arm (52) form a second electrical pathway, the traction portion (53) being configured to drive the first operating arm (51) to approach the second operating arm (52).

19. The electrocoagulation surgical instrument according to claim 18, wherein the traction portion (53) comprises a traction line (54), the traction line (54) comprising a connecting section (541) and a fixing section (542), the connecting section (541) being proximate to the first operating arm (51);
the driving component (2) further comprises a fixing portion (6), the fixing portion (6) being disposed on a side of the connecting portion (3) away from the electrocoagulation component (1) and provided with a first duct (61);
the fixing section (542) passes through the rod member (31) and extends into the first duct (61) to be fixedly connected to the fixing portion (6), the fixing portion (6) being operably moved toward or away from the connecting portion (3) to drive the first operating arm (51) to move relative to the second operating arm (52).

20. The electrocoagulation surgical instrument according to claim 19, wherein the first duct (61) is a through hole;
the traction line (54) further comprises a traction section (543);
the driving component (2) further comprises a main body (21);
an end of the connecting portion (3) away from the connecting end (311) is connected to the main body (21);
the electrocoagulation surgical instrument has an assembled state, in the assembled state, the traction section (543) extends from an end of the first duct (61) away from the connecting section (541) and pulls the sheath portion (4) to assemble with the connecting end (311), and the fixing section (542) is located within the first duct (61).

21. The electrocoagulation surgical instrument according to claim 20, wherein the rod member (31) is provided with a second duct (312), the second duct (312) being a through hole;
the main body (21) is provided with a first sliding track (211), the fixing portion (6) is slidably disposed on the first sliding track (211), the fixing portion (6) has a first position and a second position within the first sliding track (211), in the first position, the fixing portion (6) is located at an end of the first sliding track (211) proximate to the sheath portion (4) and the first duct (61) and the second duct (312) extend along the same axis, in the second position, the fixing portion (6) is located at an end of the first sliding track (211) away from the sheath portion (4), the first operating arm (51) approaches the second operating arm (52) and maintains a predetermined distance.

22. The electrocoagulation surgical instrument according to claim 21, wherein a port on a side of the first duct (61) opposite to the second duct (312) has a predetermined distance;
the fixing portion (6) is provided with a guiding tapered surface (66), a small-diameter end of the guiding tapered surface (66) being connected to a port of the first duct (61) proximate to the second duct (312).

23. The electrocoagulation surgical instrument according to claim 21, wherein the fixing portion (6) further comprises:
a positioning member (62);
a fixing screw (63); and
a base (64), provided with the first duct (61) and a side hole (641), the side hole (641) communicates with the first duct (61) and faces a lateral direction of the first duct (61), the side hole (641) has a threaded section (6411) and a sliding section (6412) in an axial direction, the sliding section (6412) is adjacent to the first duct (61), the positioning member (62) is slidably disposed in the sliding section (6412);
the fixing screw (63) is configured to pass through the threaded section (6411) and push the positioning member (62) to move into the first duct (61) to fix the fixing section (542).

24. The electrocoagulation surgical instrument according to claim 23, wherein the main body (21) is provided with a window (212), the window (212) penetrates a side wall of one side of the first sliding track (211) and has a length direction parallel to the axial direction of the first sliding track (211);
a head of the fixing screw (63) extends out from the window (212), and in the first position and the second position, the fixing screw (63) is moved to edge positions along the length direction of the window (212).

25. The electrocoagulation surgical instrument according to claim 23, wherein the base (64) and the fixing screw (63) are insulating members;
the positioning member (62) comprises a plurality of connecting handles (621) and a sliding body (622) adapted to the sliding section (6412), the plurality of connecting handles (621) protrude from a side of the sliding body (622) away from the fixing screw (63);
the fixing portion (6) further comprises:
a plurality of first elastic members (67); and
a first electrical contact member (65), disposed on the base (64) and comprising an electrical contact surface (651), the electrical contact surface (651) protruding into the first duct (61);
a bottom of the side hole (641) is provided with a plurality of first positioning holes (6413), the plurality of first positioning holes (6413) are arranged on both sides of the first duct (61), the plurality of first elastic members (67) are respectively disposed within the plurality of first positioning holes (6413);
the fixing screw (63) is screwed into the threaded section (6411), a tail of the fixing screw (63) pushes the sliding body (622) toward the electrical contact surface (651) to clamp the fixing section (542), and the first electrical contact member (65) is electrically connected to the fixing section (542), while the plurality of connecting handles (621) are respectively inserted into the plurality of first positioning holes (6413) and abut the plurality of first elastic members (67).

26. The electrocoagulation surgical instrument according to claim 21, wherein the first sliding track (211) has a tail port (2111), the tail port (2111) is located on a side of the first sliding track (211) away from the sheath portion (4);
the driving component (2) further comprises a sealing member (22), the sealing member (22) being an insulating member and comprising a limit protrusion, the limit protrusion being provided with a third duct (221);
the fixing section (542) is located within the first duct (61), the traction section (543) extends out through the tail port (2111) to an exterior of the main body (21);
the sealing member (22) seals the tail port (2111), the third duct (221) and the first duct (61) extend along the same axis, and at least part of the traction section (543) is retractably passed through the third duct (221);
in the second position, the limit protrusion abuts the fixing portion (6).

27. The electrocoagulation surgical instrument according to claim 21, wherein the connecting portion (3) comprises a first connector (32), the first connector (32) being disposed on an end of the rod member (31) away from the connecting end (311);
the main body (21) comprises:
a containing portion (23), provided with the first sliding track (211); and
a second connector (24), protruding from a side of the containing portion (23) and provided with a fourth duct (241), the traction line (54) is operably passed through the fourth duct (241) and the first sliding track (211);
the driving component (2) further comprises a first wire (25);
the first connector (32) and the second connector (24) are in a connected state, the fourth duct (241) and the first duct (61) extend along the same axis, and one end of the first wire (25) is electrically connected to the first connector (32), and the other end extends to an exterior of the main body (21).

28. The electrocoagulation surgical instrument according to claim 27, wherein the driving component further comprises:
a second electrical contact member (26), provided with a central through hole (261) that is sleeved on the traction line (54);
the second connector (24) is provided with a fifth duct (242), the fifth duct (242) communicates with the first sliding track (211);
the first connector (32) and the second connector (24) are paired and in a connected state, the second electrical contact member (26) abuts between an end surface of the rod member (31) and the second connector (24), and the central through hole (261) aligns with the first duct (61) and the fourth duct (241), the first wire (25) passes through the first sliding track (211) and the fifth duct (242) to connect to the second electrical contact member (26).

29. The electrocoagulation surgical instrument according to claim 20, wherein the traction portion (53) further comprises a moving head (531), the moving head (531) being connected to the connecting section (541) and a tail end of the first operating arm (51);
the sheath portion (4) comprises a second elastic member (41), a second sliding track (42), a stop ring surface (43), and a first containing hole (44), the first containing hole (44) being a through hole, the stop ring surface (43) being located between the second sliding track (42) and the first containing hole (44), the moving head (531) is slidably disposed within the second sliding track (42), and the second elastic member (41) abuts between the moving head (531) and the stop ring surface (43);
in a free state of the traction portion (53), the second elastic member (41) pushes the first operating arm (51) away from the second operating arm (52);
in the assembled state, at least part of the connecting end (311) is inserted into the first containing hole (44).

30. The electrocoagulation surgical instrument according to claim 29, wherein the connecting end (311) comprises a contact ring surface (3111) and a puncture head (3112), the puncture head (3112) protruding from a central region of the contact ring surface (3111);
the sheath portion (4) further comprises:
an elastic sleeve (45), disposed on the second sliding track (42), the second elastic member (41) abuts against the stop ring surface (43) via the elastic sleeve (45), the elastic sleeve (45) is provided with a tapered hole (451), a small-diameter end of the tapered hole (451) is away from the stop ring surface (43), and an inner diameter of at least part of the tapered hole (451) is smaller than an outer diameter of the puncture head (3112);
in the assembled state, the puncture head (3112) is simultaneously inserted through the first containing hole (44) and the tapered hole (451) until the contact ring surface (3111) abuts the sheath portion (4).

31. The electrocoagulation surgical instrument according to claim 30, wherein the rod member (31) is a conductive member and comprises a transition segment (313) and an opposite end (314), the transition segment (313) being located between the connecting end (311) and the opposite end (314);
the connecting portion (3) further comprises a first insulating coating layer (33), the first insulating coating layer (33) coats the transition segment (313), and the connecting end (311) is exposed outside the first insulating coating layer (33);
the sheath portion (4) further comprises a conductive body (46) and a second insulating coating layer (47) covering partial outer regions of the conductive body (46), the second operating arm (52) is disposed on the conductive body (46), the conductive body (46) has a match end surface (461);
the match end surface (461) is exposed outside the second insulating coating layer (47) and abuts the contact ring surface (3111).

32. The electrocoagulation surgical instrument according to claim 29, wherein the sheath portion (4) further comprises:
a conductive body (46), provided with a second containing hole (462), the stop ring surface (43), and the first containing hole (44), the second containing hole (462) has an opening end (4621) away from the first containing hole (44); and
an insulating liner (48), comprising a flanged edge (481) and a tubular body (482), an inner wall of the tubular body (482) forms the second sliding track (42);
the tubular body (482) is disposed within the second containing hole (462), the flanged edge (481) is clamped at the opening end (4621).

33. The electrocoagulation surgical instrument according to claim 32, wherein the moving portion (5) further comprises an insulating spacer block (56), the insulating spacer block (56) being disposed between the first operating arm (51) and the second operating arm (52);
the flanged edge (481) comprises two pieces extending in opposite directions;
the conductive body (46) comprises two support arms (464), the two support arms (464) protrude from the opening end (4621) and are staggered with the two flanged edges (481);
the insulating spacer block (56) has a first hinge hole (B1) and a second hinge hole (B2), the second operating arm (52) has a third hinge hole (B3) and a fourth hinge hole (B4), the first operating arm (51) has a fifth hinge hole (B5) and a sixth hinge hole (B6), the support arms (464) have a seventh hinge hole (B7), the moving head (531) has an eighth hinge hole (B8);
the first hinge hole (B1) and the third hinge hole (B3) are simultaneously hinged with the seventh hinge hole (B7), the second hinge hole (B2) and the fourth hinge hole (B4) are simultaneously hinged with the fifth hinge hole (B5), the eighth hinge hole (B8) is hinged with the sixth hinge hole (B6), and the two flanged edges (481) correspond to the first operating arm (51) and the second operating arm (52), respectively.

34. The electrocoagulation surgical instrument according to claim 19, wherein the traction line further comprises:
a conductive core (544) and a third insulating coating layer (545), the third insulating coating layer (545) coats the conductive core (544) and corresponding regions of the rod member (31), and the conductive core (544) and corresponding regions of the fixing portion (6) are exposed outside the third insulating coating layer (545).

35. An electrocoagulation surgical instrument, comprising:
a driving component (2), comprising a connecting portion (3), the connecting portion (3) comprising a hollow rod member (31), the rod member (31) comprising a connecting end (311); and
an electrocoagulation component (1), comprising a sheath portion (4), a traction portion (53), and a moving portion (5) disposed on the sheath portion (4), the moving portion (5) comprising a first operating arm (51) and a second operating arm (52);
the traction portion (53) is configured such that one end is connected to the moving portion (5), the other end is operably passed through the rod member (31) and pulls the sheath portion (4) to move toward the rod member (31) until the connecting end (311) is detachably connected to the sheath portion (4), the traction portion (53) being configured to drive the first operating arm (51) to approach the second operating arm (52);
the moving portion (5) is configured such that the first operating arm (51) and the second operating arm (52) are conductively connected through the sheath portion (4) and the rod member (31), and/or the first operating arm (51) and the second operating arm (52) are conductively connected with the traction portion (53).

36. The electrocoagulation surgical instrument according to claim 35, wherein the traction portion (53) is configured such that one end is connected to the first operating arm (51), the second operating arm (52) is disposed on the sheath portion (4), the first operating arm (51) and the second operating arm (52) are pivotally connected and in electrical contact;
the traction portion (53) comprises a traction line (54), the traction line (54) comprising a connecting section (541) and a fixing section (542), the connecting section (541) being proximate to the first operating arm (51);
the driving component (2) further comprises a fixing portion (6), the fixing portion (6) being disposed on a side of the connecting portion (3) away from the electrocoagulation component (1) and provided with a first duct (61);
the fixing section (542) passes through the rod member (31) and extends into the first duct (61) to be fixedly connected to the fixing portion (6), the fixing portion (6) being operably moved toward or away from the connecting portion (3) to drive the first operating arm (51) to move relative to the second operating arm (52).

37. The electrocoagulation surgical instrument according to claim 36, wherein the first duct (61) is a through hole;
the traction line (54) further comprises a traction section (543);
the driving component (2) further comprises a main body (21);
an end of the connecting portion (3) away from the connecting end (311) is connected to the main body (21);
the electrocoagulation surgical instrument has an assembled state, in the assembled state, the traction section (543) extends from an end of the first duct (61) away from the connecting section (541) and pulls the sheath portion (4) to assemble with the connecting end (311), and the fixing section (542) is located within the first duct (61).

38. The electrocoagulation surgical instrument according to claim 37, wherein the connecting portion (3) comprises a first connector (32), the first connector (32) being disposed on an end of the rod member (31) away from the connecting end (311), the rod member (31) is provided with a second duct (312), the second duct (312) being a through hole;
the main body (21) comprises:
an containing portion (23), provided with a first sliding track (211), the fixing portion (6) is slidably disposed on the first sliding track (211), the fixing portion (6) has a first position and a second position within the first sliding track (211), in the first position, the fixing portion (6) is located at an end of the first sliding track (211) proximate to the sheath portion (4) and the first duct (61) and the second duct (312) extend along the same axis, in the second position, the fixing portion (6) is located at an end of the first sliding track (211) away from the sheath portion (4), the first operating arm (51) approaches the second operating arm (52); and
a second connector (24), being a conductive member and provided with a fourth duct (241), the traction line (54) is operably passed through the fourth duct (241) and the first sliding track (211);
the first connector (32) and the second connector (24) are fixedly connected, and the second connector (24) is conductively connected to the sheath portion (4) through the rod member (31).

39. The electrocoagulation surgical instrument according to claim 38, wherein the second connector (24) is provided with a positioning groove (243), the positioning groove (243) is recessed from an outer surface of the second connector (24) toward the fourth duct (241);
the driving component (2) further comprises a housing portion (291) and a conductive ring (281), the conductive ring (281) is disposed in the positioning groove (243) and electrically connected to the second connector (24), at least part of the second connector (24) is disposed inside the housing portion (291), the housing portion (291) being an insulating member.

40. The electrocoagulation surgical instrument according to claim 39, wherein the driving component (2) further comprises a conductive arm (282), one end of the conductive arm (282) is connected to the conductive ring (281), and the other end extends to an exterior of the housing portion (291).

41. The electrocoagulation surgical instrument according to claim 38, wherein the traction portion (53) further comprises a moving head (531), the moving head (531) being connected to the connecting section (541) and a tail end of the first operating arm (51);
the sheath portion (4) comprises a second elastic member (41), a second sliding track (42), a stop ring surface (43), and a first containing hole (44), the first containing hole (44) being a through hole, the stop ring surface (43) being located between the second sliding track (42) and the first containing hole (44), the moving head (531) is slidably disposed within the second sliding track (42), and the second elastic member (41) abuts between the moving head (531) and the stop ring surface (43);
in a free state of the traction portion (53), the second elastic member (41) pushes the first operating arm (51) away from the second operating arm (52);
in the assembled state, at least part of the connecting end (311) is inserted into the first containing hole (44).

42. The electrocoagulation surgical instrument according to claim 41, wherein the connecting end (311) comprises a contact ring surface (3111) and an insertion head, the insertion head protruding from a central region of the contact ring surface (3111);
the sheath portion (4) further comprises:
an elastic sleeve (45), disposed on the second sliding track (42), the second elastic member (41) abuts against the stop ring surface (43) via the elastic sleeve (45), the elastic sleeve (45) is provided with a tapered hole (451), a small-diameter end of the tapered hole (451) is away from the stop ring surface (43), and an inner diameter of at least part of the tapered hole (451) is smaller than an outer diameter of the insertion head;
in the assembled state, the insertion head is simultaneously inserted through the first containing hole (44) and the tapered hole (451) until the contact ring surface (3111) abuts the sheath portion (4).

43. The electrocoagulation surgical instrument according to claim 42, wherein the rod member (31) is a conductive member and comprises a transition segment (313) and an opposite end (314), the transition segment (313) being located between the connecting end (311) and the opposite end (314), the first connector (32) is disposed on the opposite end (314);
the connecting portion (3) further comprises a first insulating coating layer (33), the first insulating coating layer (33) coats the transition segment (313), and the connecting end (311) is exposed outside the first insulating coating layer (33);
the sheath portion (4) further comprises a conductive body (46) and a second insulating coating layer (47) covering partial outer regions of the conductive body (46), the second operating arm (52) is disposed on the conductive body (46), the conductive body (46) has a match end surface (461);
the match end surface (461) is exposed outside the second insulating coating layer (47) and abuts the contact ring surface (3111).
